(19) Europäisches Patentamt
European Patent Office
Office européen des brevets

(11) **EP 3 078 745 B1**

(12) **EUROPEAN PATENT SPECIFICATION**

(45) Date of publication and mention of the grant of the patent:
**02.01.2019 Bulletin 2019/01**

(51) Int Cl.:
***C12Q 1/6886*** *(2018.01)*

(21) Application number: **16163071.0**

(22) Date of filing: **31.03.2016**

(54) **METHOD FOR OBTAINING INFORMATION RELATING TO OVARIAN CANCER AND OVARIAN CANCER DETECTION KIT**

VERFAHREN ZUR GEWINNUNG VON INFORMATIONEN BEZÜGLICH EIERSTOCKKREBS UND EIERSTOCKKREBSNACHWEISKIT

PROCÉDÉ POUR OBTENIR DES INFORMATIONS RELATIVES À UN CANCER DE L'OVAIRE ET KIT DE DÉTECTION DU CANCER DE L'OVAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.04.2015 JP 2015079254**

(43) Date of publication of application:
**12.10.2016 Bulletin 2016/41**

(73) Proprietors:
- **SYSMEX CORPORATION**
  **Kobe-shi,**
  **Hyogo 651-0073 (JP)**
- **The University of Tokyo**
  **Bunkyo-ku,**
  **Tokyo 113-8654 (JP)**

(72) Inventors:
- **Tai, Kaya**
  **Kobe-shi, Hyogo, 651-0073 (JP)**
- **Nagae, Genta**
  **Tokyo, 113-8654 (JP)**
- **Aburatani, Hiroyuki**
  **Tokyo, 113-8654 (JP)**

(74) Representative: **Paemen, Liesbet R.J. et al**
**De Clercq & Partners**
**Edgard Gevaertdreef 10 a**
**9830 Sint-Martens-Latem (BE)**

(56) References cited:
**EP-A1- 2 481 813**

- **Daniel Steinbach: "GENOMWEITE METHYLIERUNGSANALYSEN UND IDENTIFIZIERUNG PROGNOSTISCHER UND PRÄDIKTIVER MARKER FüR DAS Epitheliale Ovarialkarzinom", , 2 September 2014 (2014-09-02), XP055286607, Retrieved from the Internet: URL:http://d-nb.info/1064611060/34 [retrieved on 2016-07-07]**
- **DIANA L. KOLBE ET AL: "Differential Analysis of Ovarian and Endometrial Cancers Identifies a Methylator Phenotype", PLOS ONE, vol. 7, no. 3, 5 March 2012 (2012-03-05), page e32941, XP055267312, DOI: 10.1371/journal.pone.0032941**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 078 745 B1

SEQUENCE LISTING

<110> SYSMEX CORPORATION
THE UNIVERSITY OF TOKYO

<120> Method for obtainig information relating to ovarian cancer and
ovarian cancer detection kit

<130> SYSM-108-EP

<150> JP 2015-079254
<151> 2015-04-08

<160> 12

<170> PatentIn version 3.5

<210> 1
<211> 4131
<212> DNA
<213> Homo sapiens

<400> 1

gttggccagg ctggtctcga actcccgacc tcaggtgatc cgcccacctc ggcctcccaa 60
agtgctggga ttacagacgt gagccaccat gccccaccat aatgtagagt ttaaaaagca 120
atttccagaa tagtacaatg tatcaattat tgagagtaag agaatgggga caagcataga 180
cagctgtttt gagaagttct gctgtgaaat ggtagggatt gtaatagctg gaagtgggag 240
tatagtggct aaggagagtt ataattattt ttttaaataa atattactac agcttgtttg 300
caaatgggtg ggaatgatct ggtagacaga atgaaaatca taaagagcaa aatccttggg 360
aaggtggaag cttgggctcc aaagcacaga aggaggatgc tactatattt taggcagcta 420
aatggtaagt ggtcatggga tggaatgaag gcgctgaatt tgtaggctgt tgaggaggta 480
gaaccttcaa gaagtaatgc taattggatg tctggtgaca ttataaaggg gagtctggtt 540
aaaaggcagg acaaaggcca gatgcagtgg ctcatgcctg taatcctagc actttgggaa 600
gccgaggcag gtggatcacc tgaagtcagg agttcgagac cagcctggcc aacatggcga 660
aacccccctc tactaaaaat acaaaattag ctgggcgtgg ttcgggtgcc cgtgatccca 720
gctacttggg aggctgagga gggagaatcg cttgagcctg ggaggcagag gttgcagtga 780
gccgagattg tgccactgca ctccagcctg gcgacagagc aagactctgt ctcaaaaaaa 840
aaaaaaaaaa aaaaaaaagc aagacaaaag gacttctctc ctgtcacaga ctctgccctt 900
tctgagagtc tgggacccta ctattgtcac tcctaaattt tgtttgtttg tttgtttgtt 960
tgtttgtttg tttgtttttg agacagagtt tcactctgtc gcccaggctg gagtgcagtg 1020
gtgtgttctc ggctcactgc aagccctgcc tcccgggttc acgccattct cctgcctcag 1080
cctccccagt agctgggacc acaggcgccc gccaccacgc ccggctaatt ttttgtattt 1140
ttagtagaga tggggtttca ccgtgttagc caggatggtc tcgatctcct gacctcgtga 1200

tctgcccgcc tcggcctccc aaagtgctgg gattacagac gtgagccatc gcgcccagtc 1260
tgtcactcct aacttctaat ctgctctagg gagagtccca atagtccatc ttgcatactg 1320
aagattaagt gaatttcaca gccaacacca cacaatacct tctctattga gctgtaagct 1380
gcctctggac tggatggtgt tttgttttta tttttaccgt cagcaccagt gcctctcaca 1440
tagaatttag ccaagccggt taggtgttaa gtgctgctgc tgcgctgtca gaagggcaca 1500
cggggctggt tctagaccgg cctctgctgc ttactcgttc agtaatcttc tacagcaagt 1560
tcttgaactt ctctgggcct gttttctacc tcgccgagtg gttaactgcg tttaggatct 1620
tggaagcacc taagcacgaa aagcacatag gccagaagtt aagatgtccc ttggggacgc 1680
aaataccggg aatttctccg gctgttccca ccgatcaagc accagggctc tcaggtctag 1740
ctccttcgtc tccccacccc cacatatacc ctcacttttt cttcctttcc ctatgcctcg 1800
cagtccctca acagggtctg cggtcagacg tcactcccag gtgactggca atcagggatg 1860
ccgcctcgct cacctggcca cccttgaaga ttgtggatcc tgcaccggct agccggctgg 1920
cagagggcgc gccaacagcc gccagccgaa gtgcccccgc gtaggtggga ggagtgactg 1980
cctctcacca tcccccgggg atgcacctgt ggagagacgg cccgagagtt ccagggtgag 2040
tgcgcagagg agcttatctg cctgcctgcg ggtcggtgtg tcctgccggc tgctgggcgc 2100
tggcctgcgt cgcctgcttg tctcgctccc tgtccctcgt ccgctacccc ctctgccccc 2160
cctccctcca gcagccggcc tctcggtcat cccgccgggt gggtgcctgc gaacctcact 2220
cctccctcct ctgcaggagc caactggcga gccgcaggcc gccgggctcg gcctgggagc 2280
ccataaaacc cgcccgagcc gcgccgctgc gctccagccg ctgctcacca ggcagagcga 2340
gcgggcttgg ctggggcttc ctgccctgag cgcgacaccg actgccccgg accgcgcctc 2400
caagcaggct gaagggcttc cgctcttggc ttccagaaaa gtttggagaa agagaatttg 2460
aggcggattg gagggtggta gcccctcccc agccttcttt ccctcccaga agcctcactc 2520
tgcacagcgt cccccattct tcccgtcctg attccccatc ttcctgaccc ctccttctcc 2580
ctctcctggg tcgatcccag tcacattttc tccttccgaa tctcatcctc cctcctcctc 2640
tctatcccag tcctctgaac gatttccgcc tatttgcaag ccttctccct gtcattctca 2700
acgcttctcc tttctctcca cctcccctgc cactccattt tatccatcaa acctctccac 2760
ttgcatccac accctccctt catccttccc tcccagcaaa ccttgctcat ggattctggg 2820
cctctgtggg atgccaaccc cacccctcgg ggcaccctct ctgcccccaa tgccacaaca 2880
ccctggctgg gccgggatga ggagctggcc aaggtggaga tcggagtcct ggccactgtc 2940
ctggtgctgg cgaccggggg caacctggct gtgctgctga ccctgggcca gctgggccgc 3000
aagcgctccc gcatgcacct gttcgtgctg cacttagccc tgacagacct ggccgtggcg 3060
ctcttccagg tgctgccaca gctgctgtgg gacatcacct accgcttcca gggccccgac 3120

ctcctgtgca gggccgtcaa gtacctgcag gtgctcagca tgtttgcctc cacctacatg 3180

ctgctggcca tgacgctgga ccgctacctg gctgtctgtc accccctgcg cagcctccag 3240

cagccaggcc agtccaccta cctgctcatc gctgctccct ggctgctggc cgccatcttc 3300

agcctccctc aagtcttcat tttttccctg cgggaggtga tccagggctc aggggtgctg 3360

gactgctggg cagacttcgg cttcccttgg gggccacggg cctacctcac ctggaccacc 3420

ctggctatct tcgttctgcc ggtgaccatg ctcacggcct gctacagcct catctgccat 3480

gagatctgta aaaacctaaa agtcaagaca caggcctggc gggtgggagg agggggctgg 3540

aggacttggg acaggccctc accttccacc ttagctgcca ccactcgggg gctgccatct 3600

cgggtcagca gcatcaacac catctcacgg gccaagatcc gaacagtgaa gatgaccttt 3660

gtcatcgtgc tggcctacat cgcttgctgg gctcccttct tcagtgtcca gatgtggtcc 3720

gtgtgggaca agaatgcccc tgatgaaggc aagtggggtc tatgtggggg cagtgaggtg 3780

ggagagacag aaagagagga tgggggatta ggtcagggtt acaatgcctc ccagggccag 3840

gcaggtgaca aactagtggg gcaatgaagg aagatggtgc ctgctccaag tgaaaagggg 3900

cagttatgat ttccattcag cctaccgatg ccatgtcagt atcttttacg tgaaatttcc 3960

agctttaaaa attttggtaa ctaacaattt taaaatacta ggtggtacta atgaaatatg 4020

accattagca catttggcca ccaactacta gtttgctaga tgggctttaa aatcatagaa 4080

tgatagcaat ttgtggccca tgaacttgct ctctcagcct tgtccctcct g 4131

<210> 2
<211> 4110
<212> DNA
<213> Homo sapiens

<400> 2

```
aggctgagat gggagaattg cttgagccca gaagacagag gtggcagtga gctgatatca      60
tgccactgca ctgcagcctg ggtgacagag tgagacccca tctcaaagaa aaagaacaag     120
tctaagatct tggagtatac caaaacttcc gctccagggt ctctttttat ctttacttac     180
agaataactg aatcttgaca tccaagaatc tagtataatt atttgtacat agtaggtcct     240
cgatgtacgg gctgaatgaa agtgttaatt ccctatgctt ttacacgaat gtagtccaca     300
tcacctttca tctggtctat tacaacagcc taactgctct ccttcacttc agtctccatc     360
cagtccctct tcatcctgct cttggtgatg acactaccct cttcaaaacc cttggaggtc     420
tctctgctgc attcatgata aagtccaaac tccatatcct ggtattcaag gtataatata     480
ccttatttta aagtttcctc attgtaccct ttcctgctgc ctctatacac ccgctgcccc     540
aactccttac acaaagcgat gctagtccac tacctccagg aagcctttcc tggctgttag     600
cccacacaga ccttcctcta aaatagcaat gcttagcagg cctcttcctt ggctattctc     660
```

ctaaaaggcc tcacttgatt cagctttttc tggtctaggc ccacaggagc tcttcctccc 720
ctgtctcctc tcaacttggc tgggggctca gccagtaatg ggatggtggt atgtacagca 780
gtggtagggg agggagaagg ggaaagggag attcagtaaa ctgtcaattg ttttaggggg 840
aagaggccac acgggaagaa gttcctccag agccttaccc cttttccctc caacctgctc 900
ccctctagtt agccatctct tcttgcagcc acaggaattc tcatccccag ttacccacaa 960
actcctctct tcctttctcc attcttctta agccgggatt ttccttgctt ctttctagcc 1020
tgcaccttta atgctgctgc ttcccaacct gggcctccgc acccagtcat cttccttttt 1080
agccagtctt agcatcccag aagctccagg cggaaaagcc agaccctctc tgccttcttc 1140
cccatgccct aggacttttc tgagctccct gctcagctct acggagcctt tcctcctgtt 1200
ccccatgggt cggcctgccc cttgtcttct cagccgctgg ctggcctggg gcctccactg 1260
tcccctccgc acaccggccg acttctaaaa gcctcttcca ccaccactat gcaggacact 1320
caggcctcat ggtactgggc tccactccca cagccaaggg cctccccgac caccctccag 1380
cttatcccgg agcttcagtc cccccaaact ccagggcctt ttccccacac cccctcctca 1440
tggcataact caaactccgc gccctgcaac ctcgagtcct caaagtcctc actcctgtcc 1500
tgaccagggc cttcctcctg caaagacgcc acggccaccc gcaaggcctc ctggtggaac 1560
ctcccccgcg ccgcttccag gcccggggcc tgctctcctg ccccccctccc cagcaggcct 1620
cggccgggcc tgccacctct tcgcccaact ccaaccccgg cctcccgacc ccaccctcac 1680
agagccagca gcctctcagg cccgaggcct cccctgtccc cagcgcggct aggcctgctt 1740
ggcagagcct cactttcccc tctttcactt gccccacgtc ggacccaagt ttccctcgtc 1800
agcggccagg ccgcggccag cggtccccaa cccacagccc cgcctcccag cccaactggc 1860
cggttcgccc tccccggccg ttcatcccgg cgccccaggc cgccctccat ccaggcccgg 1920
ccctttggtc ccggccgcct tcaggcctgg ggcccccgtt cactcccatt cagcccaagg 1980
cctcttggcg gcgccgcgcc ccctgccacg gcagcccggc agtccgggat ccccgggccg 2040
tcgccccgct tggggcctcc ttggcccttc ccgcctgtcc gtcattcgag cctccctcgc 2100
ttgtttaagc cgctccgggc ccccctccac tcgctctccg gtccctcctc gccgctcgag 2160
ccgatccgag tggcctccgg cgctccctgt cctgcgggtc gcacggtcgc tcggtcgctt 2220
agtcagtttg gcacccgaga ccccggttgt cggttcgctc ccgtcagccc tgggccgtca 2280
gacaggccgc ggcgccccga ccctttcgtt cggcctctcc ccctgcccca ttccctcgct 2340
ctccaatctg ctgtcctccc ctccgcccat cggcttggcc tccggaatct cgacccccgt 2400
ggcgtgtcag gcagatgctg gagccccggg gccatcagtc aggttcggtg gccccgggcc 2460
tggcctggcc ttgccttagg ccgggcctcc taacctcggc ccctgcccta gggcagccgg 2520

gccatcgctg cccaccggtt cgacatccga gactctcctg cccggcccag ccgagtgtca 2580
gttcgcgcgg cccacagccg caatccagcc tcagtcgccc gggcccagcc tgcttcgctc 2640
cagaccgggc ctcctggcct cgccccccgc gcccccgagc caggccgcgg tgcgggtcag 2700
cctgcccatt taggtctccg tctttactct gtccagccct gtcagtcaca aaattcattc 2760
tccgatccta cgagctggct ttcagcgccc caggctgggc cttgccccgg gcctcccggc 2820
ctcgcccccc gcgcccccg gccgctgggc cgccccttgc tcttagccag aggtagcccc 2880
tcaccccgcg acttacccca cacccgctc tccagaaccc ccatatgggc gctcaccgcc 2940
cgcccgcaca gctcgaacag ggcgggggga gcgttggggc ccgaggccga gctcttcgct 3000
ggcgccgcct cccgggacgt ggcctccatg gtcgttgccg ccgctacctc acagaaccag 3060
caactccggg cgcgccaggc ctcgggcgcc gccatcttgg ggaggtgcgc gagcccgaga 3120
gtgtcgcccg cggaccgcca tcttgaaaag gtcagcagtt aggacggctc cataagcgat 3180
atggggaaga atgtgaatta ttctaaagaa atttcgaaga aattagcaca ctttccaagt 3240
ctcttaggag ctactatttt agataaactc ctagatcaat tatacttggg tgtggtatga 3300
ctaaggggat gtttcttagc aaagcctcct cgggtgcaaa catcagctac ccctaacctc 3360
tgcctgcggc tggtagtaca tgccaatctg agcatgtgtt cgcgaccacg atgagtgtgc 3420
cgcacttccg gccagatcgc cggatttccg ctgagtgacc cttacaagtc cttcttgatc 3480
ctgaactggg ttaggtgccg ctgttgctgc tcgtgttgaa tctagaaccg tagccagaca 3540
tgggactgga ggacgagcaa aagatgctta ccgaatccgg agatcctgag gaggtaaggc 3600
aaggcgatcc actcggccct cttctctgcc ctgaagccca ggacctagcc cgccaaaaca 3660
cgcacggggc cctcttagcc cccagtttac cctctagggt ttgcatagtc gggagctcta 3720
gttccctcga cctttcccca gaattcgtcc ggtgactttg gacagataga tgactagttt 3780
ttctctgggc ctcggcttct gcattctgtg aatgtagtga aggtttgaga gcgaaaatgt 3840
tttgtggact aaagtcttgt aaaaatatga cctatgtttg cagtcagtca gatgttctga 3900
agcccgcgtt tcacagattt ggggagcaaa cagaccgcct tgccccagct caggttagga 3960
atccctacca catcttctat ggaggttcgg aagacctggc cttgtgttcc ttttctttga 4020
ggaaggtgtg tgtctttggg gacaatgaaa gaatgtacga tgtgattctg tgatggaaga 4080
gggagctctt tcccagagaa agagtgctgc 4110

<210> 3
<211> 131
<212> DNA
<213> Homo sapiens

<400> 3

atgcacctgt ggagagacgg cccgagagtt ccagggtgag tgcgcagagg agcttatctg 60

cctgcctgcg ggtcggtgtg tcctgccggc tgctgggcgc tggcctgcgt cgcctgcttg 120

tctcgctccc t 131

<210> 4
<211> 110
<212> DNA
<213> Homo sapiens

<400> 4

ccctgccacg gcagcccggc agtccgggat ccccgggccg tcgccccgct tggggcctcc 60

ttggcccttc ccgcctgtcc gtcattcgag cctccctcgc ttgtttaagc 110

<210> 5
<211> 131
<212> DNA
<213> Artificial Sequence

<220>
<223> The sequence converted with bisulfites of the amplified region in the APVR1B promotor.

<400> 5

atguauutgt ggagagacgg uucgagagtt uuagggtgag tgcguagagg aguttatutg 60

uutguutgcg ggtcggtgtg tuutgucggu tgutgggcgu tgguutgcgt cguutguttg 120

tutcgutuuu t 131

<210> 6
<211> 110
<212> DNA
<213> Artificial Sequence

<220>
<223> The sequence converted with bisulfites of the amplified region in the LRRC41 promotr.

<400> 6

uuutguuacg guaguucggu agtucgggat uuucgggucg tcguuucgut tggggguutuu 60

ttgguuuttu ucguutgtuc gtuattcgag uutuuutcgu ttgtttaagu 110

<210> 7
<211> 23
<212> DNA
<213> Artificial Sequence

<220>
<223> Forward M primer for amplification of the region in AVPR1B promotor

<400> 7
atgtatttgt ggagagacgg ttc 23

<210> 8
<211> 23
<212> DNA
<213> Artificial Sequence

<220>
<223> Reverse M primer for amplification of the region in AVPR1B promotor

<400> 8
aaaaaacgaa acaaacaaac gac 23

<210> 9
<211> 25
<212> DNA
<213> Artificial Sequence

<220>
<223> Forward M primer for amplification of the region in LRRC41 promotor.

<400> 9
ttttgttacg gtagttcggt agttc 25

<210> 10
<211> 25
<212> DNA
<213> Artificial Sequence

<220>
<223> Reverse M primer for amplification of the region in the LRRC41 primer.

<400> 10
acttaaacaa acgaaaaaaa ctcga 25

<210> 11
<211> 33
<212> DNA
<213> Artificial Sequence

<220>
<223> Forward primer for control

<400> 11
gggatattaa gtggagttat tttggtttta gtt 33

<210> 12
<211> 21
<212> DNA
<213> Artificial Sequence

<220>
<223> Reverse primer for control

<400> 12
ccctcccaac atccttccta a 21

## Description

### TECHNICAL FIELD

The present invention relates to a method for obtaining information whether or not a biological sample collected from a subject contains ovarian cancer cells. Also, the present invention relates to use of an ovarian cancer cell detection kit in the method.

### BACKGROUND

Ovarian cancer involves numerous types of tumors because the ovary is an organ consisting of surface epithelium, germ cells, sex hormone secretory cells, and interstitial cells. Representative examples of ovarian surface epithelial cancer are serous adenocarcinoma, mucous adenocarcinoma, endometrioid adenocarcinoma, clear cell adenocarcinoma, and the like. Even young women may develop the mucous adenocarcinoma. Representative examples of ovarian germ cell cancer are dysgerminoma, yolk sac tumor, embryonic cell cancer, and the like, and most of them are observed with young women up to 35 years old. Representative examples of ovarian sex hormone secretory cell cancer are granulosa cell tumor and the like.

Ovarian cancer is known as cancer that is difficult to make an early diagnosis. One of the reasons for the difficulty is that, unlike the uterus, it is impossible to harvest cells from the ovary for examination because the ovary is located inside the peritoneal cavity. The symptoms observed with early stage ovarian cancer include abdominal distension, abdominal pains, gastrointestinal dysfunction, frequent urination, weight loss, and so forth, but these symptoms are not specific to ovarian cancer and can be observed with other diseases. This is considered as another reason for the difficulty of the early diagnosis of ovary cancer.

In recent years, methods based on gene information have been studied as new cancer diagnosis methods. Examples of these methods include methods based on information relating to methylation of DNA. In the method, a CpG site (5'-(CG)-3') in a base sequence of a predetermined gene is used as a marker, and information about the presence or absence of cancer cells and so forth is obtained based on an analysis result of a methylation state of the marker. The cancer diagnosis is performed by using the information as an index.

For instance, US 2011/0257034 A describes a method for predicting prognosis of colon cancer by measuring a methylation level of LRRC41 gene group. US 2013/0316931 A describes a method for predicting prognosis and monitoring a therapeutic effect on melanoma by measuring a methylation level of a gene group including AVPR1B. US 2010/0305058 A and US 2007/0172844 A describe methods for evaluating an effect of an anticancer drug on ovarian cancer by measuring a change in expression amount of a gene group including AVPR1B.

As described above, it has been reported that it is possible to predict the prognoses of colon cancer and melanoma by measuring a methylation level of AVPR1B gene or LRRC41 gene, but these publications neither describe nor suggest that it is possible to predict whether or not a subject suffers from ovarian cancer by measuring a methylation level of AVPR1B gene or LRRC41 gene. On the other hand, few genes can be used as markers for detecting ovarian cancer. Steinbach (2014 "Genomweite methylierungsanalysen und identifiziering prognostischer und prädiktiver marker fûr das epitheliale ovarialkarzinom") discloses that LRRC41 gene is hyper-methylated in ovarian cancer with good prognosis. Steinbach does not make the comparison with other cancer types and hence does not relate to ovarian cancer-specific diagnostic markers. Kolbe et al. (2012 PLOS One 7(3):e32941) studied the methylation status of 14495 genes in different types of gynaecologic cancer. Therefore, there is a demand for further development of a novel marker for detecting ovarian cancer by utilizing methylation analysis of a gene.

### SUMMARY OF THE INVENTION

The present inventors have identified, as novel markers, gene regions where specific methylation is confirmed in a DNA obtained from a cancerous tissue of ovarian cancer. The inventors have found that ovarian cancer-derived cancer cells can be clearly distinguished from other cells (cells of normal tissues, cells of non-cancerous tissues, and cancer cells derived from cancers except for kidney cancer and uterine cancer and different from ovarian cancer) based on results obtained by analyzing methylation states of these markers and have accomplished the present invention.

Specifically, the present invention provides a method for obtaining information whether or not a biological sample collected from a subject contains ovarian cancer cells. The method comprises the steps of analyzing methylation status of a CpG site in a promoter region of at least one gene selected from the group consisting of AVPR1B gene and LRRC41 gene contained in a DNA sample prepared from the biological sample from the subject; and obtaining information indicating that the biological sample comprises an ovarian cancer cell when it has been determined that the methylation of the CpG site in the promoter region of at least one of the AVPR1B gene and the LRRC41 gene is present in the determination step.

In the method, the promoter region may comprise a base sequence of SEQ ID NO: 3 or 4.
In the method, the analyzing step may be conducted by at least one method selected from mass spectrometry and methylation-specific PCR.
In the method, the biological sample may be a tissue, blood or serum.
In the method, the analyzing step may comprise: calculating methylation frequency of the promoter region, and the obtaining step may comprise: comparing the frequency with a predetermined threshold; and obtaining information that the biological sample comprises the ovarian cancer cell when the frequency is equal to or higher than the threshold.
In the method, a further step of preparing the DNA sample may be provided.
In the method, the preparing step may comprise: treating DNA in the biological sample with bisulfite.
The present invention provides for the use of a reagent kit in the method. The kit comprises a primer. The primer can hybridize to a promoter region of at least one gene selected from the group consisting of AVPR1B gene and LRRC41 gene. The promoter region comprises: a uracil base and a methylated cytosine base, the uracil base being converted from an unmethylated cytosine base by a bisulfite treatment.
In the use, the primer may comprise any one of base sequences of SEQ ID NOs: 7 to 10.
In the use, the primer may be used for analyzing methylation status of a CpG site in a promoter region of at least one gene selected from the group consisting of AVPR1B gene and LRRC41 gene contained in the DNA sample.
The present invention is capable of providing a method that enables obtainment of information whether or not a biological sample collected from a subject contains ovarian cancer cells. The invention is capable of providing the use of a reagent kit in the method.

BRIEF DESCRIPTION OF THE DRAWINGS

Fig. 1A is a graph showing the methylation positive rate of the promoter region of AVPR1B gene calculated from methylation data of a cancerous tissue and a non-cancerous tissue of ovarian cancer and a normal tissue.
Fig. 1B is a graph showing the methylation positive rate of the promoter region of LRRC41 gene calculated from methylation data of a cancerous tissue and a non-cancerous tissue of ovarian cancer and a normal tissue.
Fig. 2A is a graph showing the methylation positive rate of the promoter region of AVPR1B gene calculated from methylation data of various clinical specimens.
Fig. 2B is a graph showing the methylation positive rate of the promoter region of LRRC41 gene calculated from methylation data of various clinical specimens.
Fig. 3 is a photograph showing results of amplification of DNA samples extracted from ovarian cancer and normal ovary tissue by methylation-specific PCR (MSP method).
Fig. 4 is a photograph showing results of amplification of DNAs extracted from various cancer tissues and normal tissues of various organs by the MSP method.
Fig. 5 is a schematic view showing one example of a determination device for providing information relating to ovarian cancer of a subject.
Fig. 6 is a block diagram showing a functional configuration of the determination device of Fig. 5.
Fig. 7 is a block diagram showing a hardware configuration of the determination device shown in Fig. 5.
Fig. 8 is a flowchart of determination for providing information relating to ovarian cancer of a subject using the determination device shown in Fig. 5.

DETAILED DESCRIPTION OF THE PREFERRED EMBODIMENTS

In a method for obtaining information relating to ovarian cancer as provided herein (hereinafter also and simply referred to as "method"), a DNA sample obtained from a biological sample is analysed. The DNA sample may firstly be prepared from a biological sample collected from a subject.
In the methods envisaged herein, the biological sample is not particularly limited insofar as it is a sample of biological origin containing DNA of a subject. Preferably, the biological sample is a sample containing a genome DNA, such as a clinical specimen. Examples of the clinical sample include body fluid, urine, tissue collected by a surgical operation or a biopsy, and the like. Examples of the body fluid include blood, serum, plasma, lymph, abdominal dropsy, bone marrow fluid, secretory fluid from nipple, and the like. A culture obtained by culturing a cell or a tissue collected from a subject may also be used as the biological sample. A formalin-fixed paraffin-embedded (FFPE) sample of a tissue collected from a subject may also be used as the biological sample.
The DNA sample may be prepared by extracting DNA from a biological sample. The method for extracting DNA from a biological sample is publicly known in the art. For example, it is possible to extract DNA by mixing a biological sample with a treatment liquid containing a surfactant (e.g., sodium cholate, sodium dodecyl sulfate, etc.) that solubilizes a cell or a tissue and then releasing DNA contained in the biological sample into the obtained mixture liquid by performing physical treatment (stirring, homogenization, ultrasonic fragmentation, etc.) on the mixture liquid. In this case, the mixture liquid may preferably be centrifuged to allow cell debris to settle down, and a supernatant containing the released DNA may preferably be subjected to an analysis step described below. The obtained supernatant may be purified by a method publicly known in the art. The extraction of DNA from biological sample and the purification thereof may be performed by using commercially available kits.

It is preferable that the above-described preparation step further includes the step of fragmenting the extracted DNA. By forming DNA fragments having an appropriate length, it is possible to efficiently perform methylated DNA immunoprecipitation (MeDIP) and non-methylated cytosine conversion treatment described below.

The DNA fragmentation may be performed by ultrasonic treatment, alkaline treatment, restriction enzyme treatment, or the like. For example, when performing the DNA fragmentation by the alkaline treatment, DNA is fragmented by adding an aqueous sodium hydroxide solution to a DNA solution such that the final concentration becomes 0.1 to 1.0 N and then incubating the DNA solution at 10 to 40°C for 5 to 15 minutes. When performing the DNA fragmentation by the restriction enzyme treatment, a restriction enzyme to be used is appropriately selected based on a base sequence of DNA, and MseI, BamHI, or the like, for example, may be used as the restriction enzyme.

In the methods envisaged herein, the presence or absence of methylation of a CpG site present in a promoter region of AVPR1B (arginine vasopressin receptor 1B) gene or LRRC41 (Leucine Rich Repeat Containing 41) gene contained in the DNA obtained by the above-described preparation step is determined. In this specification, the "CpG site" means the site of a sequence in which cytosine (C) and guanine (G) are adjacent in this order in a direction from 5' to 3' in a base sequence. The letter "p" in CpG represents a phosphodiester bond between cytosine and guanine.

The base sequences of the promoter regions of AVPR1B and LRRC41 genes are publicly known in the art and are available from publicly known database such as the database (http://www.ncbi.nlm.nih.gov/) provided by National Center for Biotechnology Information (NCBI) of the US National Library of Medicine. ID numbers of the promoter regions of AVPR1B gene and LRRC41 gene are shown in Table 1. The base sequences of the promoter regions of AVPR1B gene and LRRC41 gene are indicated as SEQ ID NO: 1 and SEQ ID NO: 2, respectively.

[Table 1]

| Gene name | Unigene ID | Entrez Gene ID | SEQ ID NO: |
|---|---|---|---|
| AVPR1B | HS.1372 | 553 | 1 |
| LRRC41 | HS.144941 | 10489 | 2 |

In the methods envisaged herein, the determination of the presence or absence of methylation of the CpG site may be performed based on a result of methylation analysis of the promoter region. The methylation analysis may be performed by, for example, examining whether or not cytosine of at least one of the CpG sites present in the promoter region of AVPR1B gene or LRRC41 gene is methylated. In this case, the number of the CpG sites to be analyzed may be one, but it is preferable to analyze the presence or absence of the methylation of a plurality of CpG sites. The plurality of CpG sites may be selected from a promoter region of a gene or selected from each of promoter regions of a plurality of genes.

The methylation analysis may be performed by examining the frequency of methylation in the promoter region of AVPR1B gene or LRRC41 gene. As used herein, the "frequency of methylation" can be a ratio of the number of methylated CpG sites to the number of CpG sites present in the promoter region. The object to be analyzed may be a whole of the promoter region or may be a partial promoter region that includes at least one CpG site. The object to be analyzed may contain only one CpG site, but it is preferable that the object to be analyzed contains a plurality of CpG sites. The object to be analyzed may be decided from any one of the promoter regions of the above-described genes or may be decided from promoter regions of a plurality of genes. Since the locations and the numbers of the CpG sites present in the promoter regions of AVPR1B gene and LRRC41 gene are known, the number per se of the methylated CpG sites in each of the promoter regions may be used as the frequency of methylation.

The frequency of methylation may be "methylation score" that is obtained by analyzing the methylation state of a CpG site in DNA by a mass spectrometry such as MassARRAY (registered trademark) described below. In MassARRAY (registered trademark), it is possible to calculate the methylation score based on an area ratio between a peak derived from the methylated DNA fragment and a peak derived from the non-methylated DNA fragment obtained after measurement of the DNA fragments.

In the methods envisaged herein, the frequency of methylation in each of the promoter regions of AVPR1B gene and LRRC41 gene may be calculated by a hand method or may be calculated by a machine such as a computer.

In the methods envisaged herein, the CpG site (or a predetermined range including the CpG site) in the promoter region(s) of AVPR1B gene and/or LRRC41 gene to be analyzed is not particularly limited and can appropriately be decided by those skilled in the art. The locations and the numbers of the CpG sites present in the promoter regions of these genes are known. Accordingly, the CpG site or the range to be analyzed may be decided by a routine experiment employing the known analysis method described below.

As a means for the methylation analysis, various analysis methods are publicly known in the art. In the methods envisaged herein, the analysis method to be employed is not particularly limited. Preferably, the method includes the steps of distinguishing methylated DNA from non-methylated DNA, amplifying DNA, and detecting methylated DNA and/or non-methylated DNA.

Examples of the step of distinguishing methylated DNA from non-methylation DNA include the step of performing methylation-sensitive restriction enzyme treatment, MeDIP method, non-methylated cytosine conversion treatment, and the like.

Examples of the step of amplifying DNA include the step of performing PCR, quantitative PCR, IVT (in vitro transcription) amplification, SPIA (trademark) amplification, and the like.

Examples of the step of detecting methylated DNA and/or non-methylated DNA include the step of performing electrophoresis, sequence analysis, microarray analysis, mass spectrometry, southern hybridization, and the like.

The MeDIP method is a method of concentrating methylated DNA contained in a biological sample by immunoprecipitation using an antibody specifically recognizes an anti-methylated cytosine antibody, an anti-methylated cytidine antibody, or a methylated DNA-binding protein. The methylation analysis may be performed on methylated DNA obtained by concentrating, with the MeDIP method, the methylated DNA contained in the DNA obtained by the extraction step. The methylation analysis may be performed also on an amplification product obtained by amplifying the methylated DNA with IVT amplification or the like after the concentration by the MeDIP method. Such an analysis method is called MeDIP on chip.

The non-methylated cytosine conversion treatment is a treatment including reacting DNA extracted from the biological sample and a non-methylated cytosine conversion agent to convert the non-methylated cytosine in the DNA into another base (uracil, thymine, adenine or guanine). As used herein, the non-methylated cytosine conversion agent is a substance that is capable of reaction with DNA to convert non-methylated cytosine in the DNA into another base (uracil, thymine, adenine or guanine). As the non-methylated cytosine conversion agent, bisulfites such as a sodium salt, a potassium salt, a calcium salt, a magnesium salt, and the like of hydrogen sulfite are suitably usable.

In the DNA treatment using bisulfite, the non-methylated cytosine in the DNA is converted into uracil by a deamination reaction, while such conversion of the base does not occur with the methylated cytosine. Therefore, the difference in methylation state of the CpG site in DNA is converted into the difference in base sequence (differences of C and U) by the non-methylated cytosine conversion treatment with bisulfite. The non-methylated cytosine conversion treatment with bisulfite is called "bisulfite treatment".

In the case of performing the bisulfite treatment, an amount (concentration) of bisulfite to be added is not particularly limited insofar as the amount enables to satisfactorily convert the non-methylated cytosine in DNA. For example, the final concentration in a DNA-containing solution may be 1 M or more, preferably 1 to 15 M or more, more preferably 3 to 10 M. The conditions (temperature and time) for incubation after the addition of bisulfite may appropriately be set depending on the added amount of bisulfite. For example, when bisulfite is added in a final concentration of 6 M, the incubation is conducted at 50 to 80°C for 10 to 90 minutes.

The methylation of the CpG site contained in DNA may be analyzed by subjecting the bisulfite-treated DNA to a sequence analysis and detecting the difference from the original base sequence. This method is called bisulfite sequencing.

The methylation of the CpG site may be analyzed by mass spectrometry. Specifically, the PCR amplification using bisulfite-treated DNA as a template and a primer set specific for the base sequence to be analyzed is followed by further IVT amplification of the resultant PCR product so that methylated cytosine and uracil turn into guanine (G) and adenine (A), respectively. The resultant IVT amplification product is cleaved by RNase A, and the mass difference (16 Da) between G and A among the resultant nucleic acid fragments can be detected using a MALDI-TOF (Matrix Assisted Laser Desorption Ionization - Time of Flight) type mass spectrometer, thereby analyzing the methylation of DNA. This method is called MassARRAY (registered trademark) analysis.

The site to be cleaved by RNase A in the IVT product is known to be located between an arbitrary base and uracil (U) or thymine (T) adjacent to the base. Thus, the base sequence and mass of the IVT product cleaved by RNase A can be predicted from the base sequence of the DNA used as a template. Therefore, the portion of the base sequence of the template DNA, from which the respective peaks obtained by MassARRAY (registered trademark) are derived, can be identified. For example, in the case of one methylated CpG site in a DNA fragment, the peak obtained by MassARRAY (registered trademark) is shifted, by 16 Da, to the high mass side. In the analysis of a DNA fragment having a plurality of CpG sites, for example, the peak is 32 Da shifted in the case of two methylated CpG sites in the DNA fragment, and 48 Da shifted in the case of three methylated CpG sites therein.

In the mass spectrometry such as MassARRAY (registered trademark), the methylation score of the DNA fragment which has been measured can be calculated. For example, when a DNA fragment having a predetermined sequence has an area ratio between the peak derived from non-methylated DNA fragment and the peak derived from methylated DNA fragment, in the chart obtained by analysis, of 1:3, the methylation score of this DNA fragment is 3/ (1 + 3) = 0.75. Theoretically, the methylation score is 1 when all the CpG sites possessed by the DNA fragment have been methylated, and is 0 when none of the CpG sites have been methylated.

The methylation of a CpG site may be analyzed by methylation-specific PCR (MSP) method. The MSP method is a method for analyzing the presence or absence of the methylation of a CPG site by confirming the presence or absence of a PCR product through PCR amplification of bisulfite-treated DNA with the use of a primer set described below. The MSP method employs a primer set which allows for amplification of base sequence wherein the CpG site to be analyzed has been methylated (namely, cytosine has not been converted into uracil), but which does not allow for amplification of base sequences wherein the CpG site has not been methylated (namely, cytosine has been converted into uracil). It can be understood that the CpG sites to be analyzed have been methylated when a PCR product is obtained in the MSP method using such a primer set. The MSP method can also be performed by using a primer set which does not allow for amplification of base sequence wherein cytosine at the CpG site to be analyzed has not been converted into uracil, but which allows for amplification of base sequence wherein cytosine at the CpG site has been converted into uracil. In this case, it can be understood that the CpG site to be analyzed has been methylated when no PCR product is obtained.

In the methods envisaged herein, the presence or absence of the PCR product can be confirmed, for example, by gel electrophoresis. The reaction solution after amplification may be subjected to electrophoresis in a gel to visually confirm whether or not a band derived from the PCR product exists in the gel. Alternatively, an image of the gel after electrophoresis may be obtained for confirmation by image analysis. Image analysis can be conducted, for example, by obtaining a value which represents the contrast of pixels in a region where a PCR product is expected to exist (for example, band intensity) in an image of a gel and comparing this value with a predetermined threshold. Specifically, it can be determined that the PCR product could be obtained when the value which represents the contrast of pixels is a predetermined threshold or higher, whereas it can be determined that no PCR product could be obtained when the value is lower than the predetermined threshold. The predetermined threshold for the contrast of pixels is not particularly limited. For example, the threshold may be either a value which represents the contrast of background pixels or a value twice or three times the value.

Each of the primers included in the primer set used in the MSP method can appropriately be designed by those skilled in the art depending on the base sequence containing the CpG site to be analyzed. However, the primers are preferably designed so as to include cytosine at the CpG site to be analyzed at the 3' terminal or in the vicinity of the 3' terminal of the primer.

The methylation of the CpG site may be analyzed by using a microarray. In this case, the microarray for analysis may be prepared by fixing on a substrate a nucleic acid probe which is complementary to the base sequence of the promoter region of AVPR1B gene or LRRC41 gene. Such a microarray may be prepared by a method publicly known in the art.

In the analysis using the microarray, DNA extracted from a biological sample is preferably labeled by a labeling substance publicly known in the art. Accordingly, it is preferable that the determination method further includes the step of labeling the extracted DNA. Since the labeling step enables to label all of the DNAs in the biological sample, it is advantageous to perform the labeling step after the above-described DNA amplification step. Examples of the labeling substance include a fluorescent substance, hapten such as biotin, a radioactive substance, and the like. Examples of the fluorescent substance include Cy3, Cy5, FITC, Alexa Fluor (trademark), and the like. By labeling DNA as described above, measurement of a signal from the probe on the microarray is facilitated. The method for labeling DNA with the labeling substance is publicly known in the art.

The above-mentioned signal may be an appropriate signal depending on the type of the microarray. For example, the signal may be an electric signal that is generated when there are DNA fragments hybridized to the respective probes on the microarray or may be a signal such as fluorescent light and light emission generated from the labeling substance when the DNA to be analyzed is labeled as described above. The detection of the signal may be performed by a scanner provided in an ordinary microarray measurement device. Examples of the scanner include GeneChip (registered trademark) Scanner3000 7G (Affymetrix), Illumina (registered trademark) BeadArray Reader (Illumina), and the like.

In the methods envisaged herein, when an analysis result indicating that there is the methylated CpG site is obtained from the above-described methylation analysis, it is determined that the methylation is present in the CpG site existing in the promoter region of the gene. Alternatively, when a result that the frequency of methylation obtained by the methylation analysis is equal to or higher than the predetermined threshold, it is determined that the methylation is present in the CpG site existing in the promoter region of the gene. The predetermined threshold is not particularly limited and may empirically be set based on data accumulation of various biological samples. Alternatively, the predetermined threshold may be set as follows. The frequency of methylation is firstly analyzed on DNAs respectively extracted from each of a biological sample (normal ovary tissue or cell) which is confirmed to be free from cancer cells derived from ovarian cancer and a biological sample containing cancer cells derived from ovarian cancer. Next, based on the obtained analysis result, the threshold is set from a range that is higher than the frequency of methylation of the biological sample not containing cancer cells and lower than the frequency of methylation of the biological sample containing cancer cells. Preferably, a value that enables to distinguish the biological sample not containing cancer cells from the biological sample containing cancer cells with high accuracy is set as the threshold.

In the methods envisaged herein, based on the determination result obtained in the above-described determination step, information relating to ovarian cancer of a subject is obtained. As used herein, the information relating to ovarian cancer is information relating to whether or not the biological sample collected from the subject contains ovarian cancer-derived cancer cells. Additionally or alternatively, the information may be information that can be an index of diagnosis of ovarian cancer or information that assists diagnosis of ovarian cancer and preferably is information indicating development or a condition or both of ovarian cancer in the subject. Examples of the information include the possibility of development of ovarian cancer in the subject, a risk of future development of ovarian cancer in the subject, and the like. In the case where ovarian cancer has been developed in the subject, examples of the information relating to ovarian cancer include prognosis of the subject, progress (stage) of the cancer, and the like.

In the methods envisaged herein, when it is determined that there is the methylated CpG site in the determination step, information indicating that the biological sample contains ovarian cancer-derived cancer cells is obtained. Additionally or alternatively, it is possible to obtain information suggesting development of ovarian cancer or information indicating that the condition of ovarian cancer is inferior (or bad). It is also possible to obtain information indicating that the subject has high risk of developing ovarian cancer or the information indicating ovarian cancer has already been developed. If the subject has already developed ovarian cancer, it is possible to obtain information indicating that prognosis of the subject is inferior (or bad) or information indicating that the condition of cancer is at a more progressed stage.

In contrast, when it is determined that any methylated CpG site is not present in the determination step, information indicating that the biological sample does not contain ovarian cancer-derived cancer cells is obtained. Additionally or alternatively, it is possible to obtain information suggesting that ovarian cancer is not developed or information indicating that the condition of ovarian cancer is good. It is also possible to obtain information indicating that the subject has a low risk of developing ovarian cancer or information indicating that ovarian cancer has not been developed yet. If the subject has already developed ovarian cancer, it is possible to obtain information indicating that prognosis of the subject is good or information indicating that the condition of cancer is at a stage where the cancer has not progressed very much.

The scope of the present disclosure also encompasses a nucleic acid to be used as a marker for obtaining information relating to ovarian cancer through methylation analysis (hereinafter also referred to merely as "marker"). Such a nucleic acid may be an isolated nucleic acid containing at least one selected from CpG sites present in the promoter region of AVPR1B gene or LRRC41 gene. Methylation analysis can be performed for the above-described marker in the DNA sample prepared from the biological sample collected from a subject, and information relating to ovarian cancer of the subject can be obtained based on the obtained analysis result. The methylation analysis and the obtainment of the information about ovarian cancer are the same as those described above.

The scope of the present disclosure may also encompass a nucleic acid having a sequence obtained by subjecting to bisulfite treatment a continuous base sequence of a whole or a partial promoter region of AVPR1B gene or LRRC41 gene as the nucleic acid to be used as the marker. In such a nucleic acid, the promoter region can include a CpG site and a cytosine base that does not constitute the CpG site. The cytosine base that does not constitute the CpG site may be any cytosine other than the cytosine contained in the CpG site, and examples of the cytosine include a cytosine in a base sequence (namely, CA, CT or CC) wherein cytosine (C) and adenine (A), thymine (T) or cytosine (C) are adjacent in this order in the 5' $\rightarrow$ 3' direction. Such a nucleic acid can be derived from a nucleic acid isolated from the subject.

In the uses envisaged herein, it is possible to perform methylation analysis for the above-described marker in the DNA sample prepared from the biological sample collected from a subject and to obtain information relating to ovarian cancer based on the obtained analysis result. The methylation analysis and the obtainment of the information about ovarian cancer are the same as those described above.

Where the nucleic acid is to be used as the marker, non-methylated cytosine in the above-described isolated DNA may be converted into uracil by the bisulfite treatment of the isolated DNA, but methylated cytosine may remain as it is. It is possible to obtain information relating to ovarian cancer by performing the methylation analysis of the CpG site in the nucleic acid used as the marker. It is possible to obtain the isolated DNA in the same manner as in the preparation of the DNA sample described above. The bisulfite treatment, the methylation analysis, and the obtainment of information relating to ovarian cancer are also the same as those as described above.

The size of the nucleic acid to be used as the marker is not particularly limited insofar as it enables the methylation analysis by the MSP method, sequencing, or mass spectrometry, but the size may preferably be 50 to 200 bases, more preferably 80 to 130 bases.

Examples of the nucleic acid to be used as the marker include nucleic acids to be used as a marker, which consist of any one of the base sequences of SEQ ID NOs: 3 to 6. The nucleic acids to be used as the marker, which consist of any one of the base sequences of SEQ ID NOs: 3 to 6, are suitable for methylation analysis by the MSP method.

The scope of the present disclosure also encompasses a reagent kit (hereinafter also and simply referred to as "kit") for ovarian cancer detection. The kit envisaged herein may include a primer for amplifying a bisulfite-treated nucleic acid. Such a primer is capable of specifically hybridizing to a nucleic acid comprising a base sequence wherein cytosine that is present in a site other than a CpG site has been converted into another base in a base sequence having the CpG site in the promoter region of AVPR1B gene or LRRC41 gene, and is capable of hybridizing to a region including a methylated CpG site. Preferably, the primer hybridizes to a nucleic acid comprising a base sequence wherein cytosine that is present in a site other than a CpG site has been converted into another base and comprising a methylated CpG site. Preferably, the kit comprises a first primer capable of hybridizing to a methylated CpG site in the promoter region of AVPR1B gene or LRRC41 gene

The primer included in the kit may be a primer for performing methylation analysis of the CpG site by the MSP method, an analysis method involving PCR amplification such as bisulfite sequence method, or mass spectrometry such as MassARRAY (registered trademark). Preferably, the primer may be a primer that is used in the MSP method or mass spectrometry such as MassARRAY (registered trademark). The base sequence of the primer can appropriately be set by those skilled in the art depending on the above-described base sequence of the promoter region. Examples of the primer include a primer having a base sequence represented by any one of SEQ ID NOs: 7 to 10.

The kit may also include a second primer. Such a second primer may be hybridized to a nucleic acid comprising a base sequence wherein cytosine has been converted into another base in a base sequence having a CpG site in the promoter region of AVPR1B gene or LRRC41 gene. The second primer may be hybridized to a region including a sequence wherein cytosine at a non-methylated CpG site has been converted into another base by conversion treatment.

The present disclosure also encompasses a program product for causing a computer to execute obtainment of information relating to ovarian cancer of a patient. Examples of such a program product include a program that can be downloaded via the internet or the like, a computer-readable recording medium on which the program is recorded, and the like.

There is exemplified a program for causing a computer to execute the following steps of:

obtaining from a measurement device the analysis results of methylation of a CpG site present in the promoter region of at least one of AVPR1B gene and LRRC41 gene contained in a DNA sample derived from a subject; and determining the presence or absence of ovarian cancer cells in the biological sample based on the obtained analysis results.

Hereinafter, an exemplary device which is suitable for implementing the method described herein will be described with reference to the drawings. Fig. 5 is a schematic view showing one example of a diagnosis supporting device for use in obtainment of information relating to ovarian cancer of a subject. The diagnosis supporting device 10 shown in Fig. 5 includes a measurement device 20 and a determination device 30 connected to the measurement device 20.

When methylation analysis is performed by the MSP method, the measurement device 20 can be a gel imaging device such as a fluorescent image scanner. In this case, when a reaction solution which has been subjected to nucleic acid amplification by the MSP method is subjected to gel electrophoresis, and the gel after electrophoresis is set in the measurement device 20, the measurement device 20 detects an amplification product. The measurement device 20 obtains gel image information of the amplification product and provides the obtained information to the determination device 30.

The measurement device 20 may be a MALDI-TOF type mass spectrometry device. In this case, the measurement device 20 obtains mass spectrometry information including the time of flight and mass-to-charge ratio (m/z value) of a substance to be tested. When a measurement sample prepared from a DNA sample derived from a subject is set in the measurement device 20, the measurement device 20 obtains mass spectrometry information of the nucleic acid contained in the measurement sample and provides the obtained mass spectrometry information to the determination device 30.

The determination device 30 includes a computer main body 300, an input unit 301 including a keyboard or a mouse, and a display unit 302 including an LCD or a CRT and displaying information of a specimen, determination results, and so forth. The determination device 30 obtains gel image information of the amplification product from the measurement device 20. The determination device 30 executes a program that provides information relating to ovarian cancer in a subject based on the information.

The determination device 30 may be a separate device from the measurement device 20 as shown in Fig. 5 or may be a device in which the measurement device 20 is incorporated. In the latter case, the determination device 30 may per se be the diagnosis supporting device 10.

Fig. 6 is a block diagram showing software of the computer main body 300 of the determination device 30 as functional blocks. As shown in Fig. 6, the computer is provided with an obtainment unit 321, a storage unit 322, a calculation unit 323, a determination unit 324, and an output unit 325. The obtainment unit 321 is connected, through a network, to the measurement device 20 so as to be able to communicate with the device.

The obtainment unit 321 obtains the information provided by the measurement device 20. The storage unit 322 stores formulae and/or processing programs for obtaining the threshold and band intensity required for determination therein. The calculation unit 323 calculates the band intensity in accordance with the stored processing program using the information obtained by the obtainment unit 321. The determination unit 324 determines whether or not the band intensity obtained by the obtainment unit 321 or calculated by the calculation unit 323 is equal to or higher than the threshold stored in the storage unit 322. The output unit 325 outputs the determination results from the determination unit 324 to the display unit 302 as information relating to ovarian cancer of a subject (e.g., the presence or absence of ovarian cancer cells in the biological sample collected from the subject).

Fig. 7 is a block diagram showing a hardware configuration of the computer main body 300 shown in Fig. 6. As shown in Fig. 7, the computer main body 300 is provided with a CPU (Central Processing Unit) 310, a ROM (Read Only Memory) 311, a RAM (Random Access Memory) 312, a hard disk 313, an input/output interface 314, a readout device 315, a communication interface 316, and an image output interface 317. The CPU 310, ROM 311, RAM 312, hard disk 313, input/output interface 314, readout device 315, communication interface 316, and image output interface 317 are connected by a bus 318 so as to be capable of data communication.

The CPU 310 is capable of executing a program stored in the ROM 311 and a program loaded on the RAM 312. The functions shown in Fig. 6 are executed when the CPU 310 executes the program. Thus, the determination device 30 functions as the determination device for providing the information relating to ovarian cancer of a subject.

The ROM 311 is composed of mask ROM, PROM, EPROM, EEPROM and the like. The ROM 311 has recorded therein programs to be executed by the CPU 310 as described above and data to be used for execution of these programs.

The RAM 312 is composed of SRAM, DRAM and the like. The RAM 312 is used in readout of programs recorded in the ROM 311 and the hard disk 313. The RAM 312 is also utilized as a working area of the CPU 310 when these programs are executed.

The hard disk 313 has installed therein an operating system to be executed by the CPU310, programs such as an application program (computer program for providing information relating to ovarian cancer of a subject), and data to be used for execution of the programs.

The readout device 315 is composed of a flexible disk drive, a CD-ROM drive, a DVD-ROM drive and the like. The readout device 315 can read out programs or data stored in a portable recording medium 40.

The input/output interface 314 is composed, for example, of serial interfaces such as USB, IEEE1394 and RS-232C, parallel interfaces such as SCSI, IDE and IEEE1284, and analog interfaces including, for example, a D/A converter and an A/D converter. To the input/output interface 314, the input unit 301 such as a keyboard or a mouse is connected. Operators can input various commands to the computer main body 300 by means of the input unit 301.

The communication interface 316 is, for example, an Ethernet (registered trademark) interface. The computer main body 300 can also transmit printing data, for example, to a printer by means of the communication interface 316.

The image output interface 317 is connected to the display unit 302 which is composed of LCD, CRT or the like. Thus, the display unit 302 can output a video signal in accordance with the image data given from the CPU 310. The display unit 302 displays an image (screen) in accordance with the input video signal.

Hereinafter, an exemplary processing procedure for obtaining information relating to ovarian cancer of a subject by the diagnosis supporting device 10 will be described.

Fig. 8 is one example of a flow chart for obtaining information relating to ovarian cancer. Explanation is made herein about an example case where band intensity is acquired from the gel image information obtained using a biological sample derived from a subject to determine whether or not the obtained band intensity is equal to or higher than the threshold.

First, in Step S1-1, the obtainment unit 321 of the diagnosis supporting device 10 obtains gel image information about at least one of AVPR1B gene and LRRC41 gene from the measurement device 20. Next, in Step S1-2, the calculation unit 323 obtains the band intensity from the obtained gel image information and transmits the band intensity to the storage unit 322.

After that, in Step S1-3, the determination unit 324 determines whether or not the band intensity obtained by Step S1-2 is equal to or higher than the threshold stored in the storage unit 322. The routine proceeds to Step S1-4 when the band intensity is equal to or higher than the threshold. Then, the determination unit 324 transmits to the output unit 325 determination results indicating that the biological sample derived from the subject has ovarian cancer cells. On the other hand, the routine proceeds to Step S1-5 when the band intensity is lower than the threshold. Then, the determination unit 324 transmits to the output unit 325 determination results indicating that the biological sample derived from the subject does not have any ovarian cancer cell.

Finally, in Step S1-6, the output unit 325 outputs the determination results as information relating to ovarian cancer of the subject to cause the display unit 302 to display the results. Thus, the diagnosis supporting device 10 is capable of providing a physician or the like with information that supports the diagnosis on whether or not the subject suffers from ovarian cancer.

Hereinafter, the present invention will be described in detail by way of examples. However, the present invention is not limited to these examples.

## EXAMPLES

### Example 1: Identification of Novel Marker by Utilizing Methylation Data of Cancerous Tissues and Non-cancerous Tissue of Ovarian Cancer and Normal Tissues

(1) Obtainment of Methylation Data

In Example 1, for cancerous tissues of ovarian cancer (49 specimens) and a non-cancerous tissue of ovarian cancer (one specimen), methylation data of Infinium HumanMethylation450 BeadChip (Illumina) published on TCGA (The Cancer Genome Atlas: http://tcga-data.nci.nih.gov/tcga/tcgaHome2.jsp) was obtained. For normal tissues (23 specimens), methylation data of Infinium HumanMethylation450 BeadChip published in the document of Nazor KL. et al. (Recurrent variations in DNA methylation in human pluripotent stem cells and their differentiated derivatives. Cell Stem Cell 2012;10(5):620-634) was obtained.

(2) Identification of Novel Marker

As a result of data mining using Infinium HumanMethylation450 BeadChip (Illumina), the promoter regions of AVPR1B genes or LRRC41 genes were identified as markers that were confirmed to be methylated specifically in cancerous tissue of ovarian cancer (see Fig. 1). Hereinafter, these markers are referred to as markers of this example.

### Example 2: Comparison among Methylation Data of Plurality of Types of Cancer/Tumor-Derived Cancer/Tumor Tissue Specimens, Non-Cancerous Tissue Specimens, and Normal Tissue Specimens

(1) Obtainment of Methylation Data

In Example 2, methylation data of 13 types of cancer/tumor tissue specimens, 10 kinds of non-cancerous tissue specimens, and 19 types of normal tissue specimens were compared. The numbers of the respective tissue specimens are shown in the tables below.

[Table 2]

| Cancer/tumor specimen | |
|---|---|
| Tissue | Number of specimens |
| Brain tumor | 114 |
| Lung cancer | 370 |
| Breast cancer | 548 |
| Gastric cancer | 69 |
| Colon cancer | 324 |
| Liver cancer | 81 |
| Bladder cancer | 78 |
| Ovarian cancer | 49 |
| Prostatic cancer | 153 |
| Acute leukemia | 192 |
| Sarcoma | 73 |
| Malignant melanoma | 242 |
| Thyroid cancer | 316 |

[Table 3]

| Non-cancerous tissue | |
|---|---|
| Tissue | Number of samples |
| Brain tumor | 1 |
| Lung cancer | 75 |
| Breast cancer | 98 |
| Gastric cancer | 2 |
| Colon cancer | 40 |
| Liver cancer | 14 |
| Bladder cancer | 15 |
| Ovarian cancer | 1 |
| Prostatic cancer | 49 |
| Thyroid cancer | 36 |

[Table 4]

| Normal tissue | | | | |
|---|---|---|---|---|
| Tissue | RCAST | Article 1 | Article 2 | Total |
| Normal brain (Brain) | 2 | 1 | 0 | 3 |
| Normal oral cavity (Oral) | 2 | 0 | 0 | 2 |
| Normal lung (Lung) | 0 | 2 | 0 | 2 |
| Normal colon mucosa (Colon) | 2 | 0 | 0 | 2 |
| Normal liver (Liver) | 2 | 0 | 0 | 2 |
| Peripheral blood of healthy individual (Blood) | 2 | 2 | 0 | 4 |
| Normal skeletal muscle (Skeletal) | 2 | 2 | 0 | 4 |
| Normal testis (Testis) | 1 | 0 | 0 | 1 |
| Normal stomach mucosa (Stomach) | 0 | 1 | 0 | 1 |
| Normal pancreas (Pancreas) | 0 | 2 | 0 | 2 |
| Normal spleen (Spleen) | 0 | 2 | 0 | 2 |
| Normal kidney (Kidney) | 0 | 0 | 0 | 0 |
| Normal adrenal gland (Adrenal gland) | 0 | 2 | 0 | 2 |
| Normal ureter (Ureter) | 0 | 2 | 0 | 2 |
| Normal bladder (Bladder) | 0 | 2 | 0 | 2 |
| Normal lymph nodes (Lymph nodes) | 0 | 2 | 0 | 2 |
| Normal adipose (Adipose tissue) | 0 | 2 | 0 | 2 |
| Normal heart (Heart) | 0 | 1 | 0 | 1 |
| Various normal blood cell ingredients (WB, PBMC, Gran, CD4+, CD8+, CD14+, CD19+, CD56+, Neu, Eos) | 0 | 0 | 60 | 60 |

For the specimens in the column "RCAST" in Table 4, the present inventors obtained the methylation data by the Infinium Methylation Assay using Infinium HumanMethylation450 BeadChip (Illumina). For the specimens in the columns "Article 1" and "Article 2," the methylation data of Infinium HumanMethylation450 BeadChip (Illumina) published in the following documents was obtained.

- Article 1: Nazor KL et al., Recurrent variations in DNA methylation in human pluripotent stem cells and their differentiated derivatives. Cell Stem Cell 2012; 10(5): 620-634
- Article 2: Reinius LE et al., Differential DNA Methylation in Purified Human Blood Cells: Implications for Cell Lineage and Studies on Disease Susceptibility, PLoS One, 7 (7) e41361

As used herein, the methylation data is a methylation rate (mCpG) of each of CpG regions of AVPR1B and LRRC41 obtained as follows. Infinium HumanMethylation450 BeadChip is provided with probes for methylation and probes for non-methylation for each of 482,421 CpG sites among the CpG sites in the human genome. The signal intensity (signal M) of the probes for methylation for the CpG sites of the target genes and the signal intensity (signal U) of the probes for non-methylation were detected by BeadArray Reader to calculate the methylation rates (mCpG) of the CpG regions of the target genes by the following calculation formula.

$$(\text{mCpG}) = (\text{signal M}) / \{(\text{signal M}) + (\text{signal U})\}$$

(2) Comparison among Methylation Positive Rates of Cancer/Tumor Tissue Specimens, Non-Cancerous Tissue Specimens, and Normal Tissue Specimens

When a statistically significant difference between the tumor tissue specimens and the normal tissue specimens was observed for the methylation rate (mCpG) obtained, the specimens were defined as being methylation positive. The methylation positive rates (%) for the various types of cancer were calculated.

$$\text{Methylation positive rate (\%)} = (\text{number of methylation-positive specimens} / \text{total number of specimens}) \times 100$$

(For example, in the case of cerebral tumors, the methylation positive rate is calculated by the formula: "number of methylation-positive specimens among cerebral tumor specimens / total number of cerebral tumor specimens (=114) × 100.")

The results are shown in Fig. 2. In this figure, the "normal tissue" represents normal tissues except 60 specimens of various normal blood cell ingredients among the tissues indicated in Table 4, and the "normal blood cell" represents 60 specimens of various normal blood cell ingredients. From Fig. 2, all the markers according to the present example hardly exhibit methylation in the non-cancerous tissues as well as the human normal tissues and human normal blood cells. All the markers according to the present example were found to have been particularly highly methylated in ovarian cancer as compared with other types of cancer. Accordingly, it was shown that the markers of the present example are suitable for detection of ovarian cancer.

Example 3: Comparison between Methylated Data (MSP) of Tissues from Healthy Individual and Ovarian Cancer Patients

(1) Biological Samples

In Example 3, FFPE samples (3 specimens) of cancerous tissues collected from ovarian clear cell adenocarcinoma patients, FFPE samples (3 specimens) of cancerous tissues collected from ovarian endometrioid adenocarcinoma patients, and FFPE samples (3 specimens) of cancerous tissues collected from ovarian serous adenocarcinoma patents were used as ovarian cancer patient-derived biological samples. A normal ovarian tissue (one specimen) was used as a control sample.

(2) Preparation of Measurement Sample

(i) Extraction of Genome DNA

The genome DNA was extracted from the respective FFPE samples of the respective ovarian cancer tissues described above using QIAamp DNA FFPE Tissue Kit (QIAGEN). The genome DNA was extracted from the normal tissues using QIAamp DNA Mini Kit (QIAGEN). As control genome DNA, the genome DNA of human peripheral blood lymphocytes was used. This genome DNA of human peripheral blood lymphocytes was amplified using GenomiPhi v2DNA amplification Kit (GE Healthcare Life Sciences). The resultant amplification product is composed of non-methylated DNA. Next, the amplification product was fragmented by Bioruptor (manufactured by COSMO BIO) to obtain a solution of non-methylated DNA fragments (0% methylated DNA). A part of the solution of this non-methylated DNA fragments was taken. SssI methylase (New England Biolab) was reacted with this for methylation of all the cytosines contained in the CG sequence, thereby obtaining a solution of methylated DNA fragments (100% methylated DNA).

(ii) Bisulfite Treatment

The respective DNA fragments obtained above (500 ng) were subjected to bisulfite treatment using EZ DNA Methylation Kit (Zymo Research), and the treated genome DNA was eluted in sterile distilled water (80 μl).

(3) MSP

The samples for measurement and control samples obtained in the above (2) were used for MSP. The compositions of the PCR reagents used, primer sets and PCR conditions are indicated as follows.

<PCR reagent>

| | |
|---|---|
| DW (Sterilized water) | 18.8 μL |
| 10 × PCR buffer with $MgCl_2$ (Roche) | 2.5 μL |
| 10 mM dNTP mix | 0.5 μL |
| 10 μM sense primer | 1.0 μL |
| 10 μM antisense primer | 1.0 μL |
| Faststart Taq polymerase (Roche) | 0.2 μL |
| Measurement sample | 1.0 μL |
| Total | 25.0 μL |

<Primer Set>

The primer sets used for the above-described MSP are shown in Table 5. Each of these primer sets enables to obtain an amplification product when DNA in a region to be amplified is methylated (hereinafter also referred to as "methylation detection primer set"). A primer set for determining whether or not the bisulfite treatment had been properly conducted was also used as a primer set for the management of accuracy (see Table 6). The base sequence of the region to be analyzed by the primer set for the detection of methylation in the promoter regions of AVPR1B gene and LRRC41 gene are respectively indicated as SEQ ID NOs: 3 and 4.

[Table 5]

| Gene name | Sequence | SEQ ID NO: | PCR product (bp) | Annealing temperature (X) | Cycle (Y) | Amplification region |
|---|---|---|---|---|---|---|
| AVPR1B | ATGTATTTGTGGAGAGACGGTTC | 7 | 131 | 60 | 36 | chtr1: 206, 223, 633-206, 223, 763 |
| | AAAAAACGAAACAAACAAACGAC | 8 | | | | |
| LRRC41 | TTTTGTTACGGTAGTTCGGTAGTTC | 9 | 110 | 60 | 38 | chr1: 46,767,900-46,768,009 |
| | ACTTAAACAAACGAAAAAAACTCGA | 10 | | | | |

[Table 6]

| Primer for Management of Accuracy | Base sequence | SEQ ID NO: | PCR product (bp) | Annealing temperature (X) | Cycle (Y) |
|---|---|---|---|---|---|
| Forward | GGGATATTAAGTGGAGTTATTTTGGTTTTAGTT | 11 | 129 | 60 | 40 |
| Reverse | CCCTCCCAACATCCTTCCTAA | 12 | | | |

<PCR Reaction Conditions>

Six minutes at 95°C,
Y cycles of 30 seconds at 95°C, 30 seconds at X°C, and 30 seconds at 72°C,
seven minutes at 72°C, and
left to stand still at 16°C.

In the above reaction conditions, "X" and "Y" respectively represent an annealing temperature and a number of cycles shown in Tables 5 and 6.

(4) Analysis Results of Methylation-Specific PCR (MSP)

The amplification products obtained by the above-described MSP were confirmed through 2% agarose gel electrophoresis. The results are shown in Fig. 3. In this figure, the numerical values "0" and "100," which are shown as "control," represent 0% methylated control samples and 100% methylated control samples, respectively.

From Fig. 3, bands were detected for all the samples in PCR using the primer sets for the management of accuracy. This reveals that the samples were properly bisulfite-treated. No band derived from methylated CpG was detected for the samples of normal ovarian tissues in the PCR using the primer set for detection of methylation. In contrast, in PCR of the samples of the ovarian cancer tissues, bands were detected for AVPR1B with 5 cases out of the 9 cases. As to LRRC41, bands were detected with 3 cases out of the 9 cases. Thus, the methylation analysis of the markers of the present example by the MSP method indicated the correlation between the methylation of the markers of the present example and ovarian cancer, as with the results of the Infinium method in Example 1. In other words, it was indicated that AVPR1B and LRRC41 are highly specific markers that show methylation tendencies in ovarian cancers at high rate but are not detected in normal ovarian tissues.

Example 4: Comparison among Methylation Data of Various Cancerous Tissues and Normal Tissues

(1) Biological Samples

In Example 4, tissue samples collected from various cancer patients and normal tissues of various organs were used as biological samples. The details of the samples are shown in Table 7. In Table 7, "T" represents a tumor tissue, "N" represents a normal tissue, and "PBL" represents a peripheral blood leukocyte.

[Table 7]

| Tissue of origin | | Number of specimens | Provider | Notation |
|---|---|---|---|---|
| Colon | Cancer | 2 | Purchased from BioChain Institute, Inc. | T1 |
| | | | | T2 |
| | Normal | 3 | | N1 |
| | | | | N2 |
| | | | | N3 |
| Brain | Cancer | 1 | Tokyo university | T |
| | Normal | 2 | Purchased from BioChain Institute, Inc. | N1 |
| | | | | N2 |

(continued)

| Tissue of origin | | Number of specimens | Provider | Notation |
|---|---|---|---|---|
| Lung | Cancer | 1 | Purchased from BioChain Institute, Inc. | T |
| | Normal | 2 | Tokyo university | N1 |
| | | | | N2 |
| Lacteal gland | Cancer | 1 | Purchased from BioChain Institute, Inc. | T |
| | Normal | 2 | | N1 |
| | | | | N2 |
| Liver | Cancer | 1 | Purchased from BioChain Institute, Inc. | T |
| | Normal | 2 | Tokyo university | N1 |
| | | | | N2 |
| Kidney | Cancer | 1 | Purchased at BioChain Institute, Inc. | T |
| | Normal | 2 | Tokyo university | NI |
| | | | | N2 |
| Uterine body | Cancer | 1 | Tokyo university | T |
| | Normal | 2 | | N1 |
| | | | | N2 |
| Bladder | Cancer | 1 | Purchased from BioChain Institute, Inc. | T |
| | Normal | 1 | | N |
| Uterine cervix | Normal | 1 | Purchased from BioChain Institute, Inc. | Cervix |
| Testis | Normal | 1 | Purchased from BioChain Institute, Inc. | Testis |
| Blood cell | Normal | 1 | Purchased from BioChain Institute, Inc. | PBL |
| Stomach | Cancer | 1 | Purchased from BioChain Institute, Inc. | T |
| | Normal | 1 | | N |

(2) Preparation of Measurement Samples and Control Samples

(i) Extraction of Genome DNA

The genome DNA was extracted from the respective tissue samples in the same manner as in Example 3. As control genome DNA, the genome DNA of human peripheral blood lymphocytes was used. A solution of non-methylated DNA fragments (0% methylated DNA) and a solution of methylated DNA fragments (100% methylated DNA) were obtained from the genome DNA of human peripheral blood lymphocytes in the same manner as in Example 3.

(ii) Bisulfite Treatment

The respective DNA fragments obtained above (500 ng) were subjected to bisulfite treatment using EZ DNA Methylation Kit (Zymo Research), and the treated genome DNA was eluted in sterile distilled water (80 μl).

(3) MSP

The samples for measurement and control samples (DNA after bisulfite treatment) obtained in the above (2) were used for MSP. The primer sets, the composition of the PCR reagent, and the PCR reaction conditions used in the MSP of Example 4 are the same as those in Example 3.

(4) Analysis Results

The amplification products obtained by the above-described MSP were confirmed through 2% agarose gel electrophoresis. The results are shown in Fig. 4. In this figure, "NC" and "PC" represent 0% methylated control samples and 100% methylated control samples, respectively. Further, "Management of Accuracy" represents an amplified product of a primer for the management of accuracy.

From Fig. 4, bands were detected for all the samples in PCR using the primer sets for the management of accuracy. Accordingly, this reveals that the samples were properly bisulfite-treated. No band derived from methylated CpG was detected with the samples of any cancerous tissue and normal tissue in the PCR using the primer set for detection of methylation. These results suggested that the markers of the present example were ovarian cancer-specific markers.

## Claims

1. A method for obtaining information whether or not a biological sample collected from a subject contains ovarian cancer cells, the method comprising the steps of:

   analyzing methylation status of a CpG site in a promoter region of at least one gene selected from the group consisting of AVPR1B gene and LRRC41 gene contained in a DNA sample prepared from the biological sample of the subject; and
   obtaining information indicating that the biological sample comprises an ovarian cancer cell when it has been determined that the methylation of the CpG site in the promoter region of at least one of the AVPR1B gene and the LRRC41 gene is present in the determination step.

2. The method according to claim 1, wherein the promoter region comprises a base sequence of SEQ ID NO: 3 or 4.

3. The method according to claim 1 or 2, wherein analyzing step is conducted by at least one method selected from mass spectrometry and methylation-specific PCR.

4. The method according to any one of claims 1 to 3, wherein the biological sample is a tissue, blood or serum.

5. The method according to any one of claims 1 to 4, wherein
   the analyzing step comprises: calculating methylation frequency of the promoter region, and
   the obtaining step comprises: comparing the frequency with a predetermined threshold; and obtaining information that the biological sample comprises the ovarian cancer cell when the frequency is equal to or higher than the threshold.

6. The method according to any one of claims 1 to 5, which further compriess the step of preparing the DNA sample from the biological sample.

7. The method of claim 6, wherein the preparing step comprises: treating DNA in the biological sample with bisulfite.

8. Use of a reagent kit in a method for obtaining information whether or not a biological sample collected from a subject contains ovarian cancer cells according to claim 1, said kit comprising a primer, wherein the primer can hybridize to a promoter region of at least one gene selected from the group consisting of AVPR1B gene and LRRC41 gene, wherein the promoter region comprises: a uracil base and a methylated cytosine base, the uracil base being converted from a unmethylated cytosine base by a bisulfite treatment.

9. Use according to claim 8, wherein the primer comprises any one of base sequences of SEQ ID NOs: 7 to 10.

10. Use according to claim 8 or 9,
    wherein the primer is used for analyzing methylation status of a CpG site in a promoter region of at least one gene selected from the group consisting of AVPR1B gene and LRRC41 gene contained in the DNA sample.

## Patentansprüche

1. Verfahren zum Erhalten von Informationen, ob eine aus einem Individuum entnommene biologische Probe Eierstockkrebszellen enthält oder nicht, wobei das Verfahren die Schritte umfasst:

   Analysieren des Methylierungszustands einer CpG-Stelle in einer Promotorregion von mindestens einem Gen, ausgewählt aus der Gruppe bestehend aus dem AVPR1B-Gen und dem LRRC41-Gen, die in einer aus der biologischen Probe eines Individuums präparierten DNA-Probe enthalten sind; und
   Erhalten von Informationen, die anzeigen, dass die biologische Probe eine Eierstockkrebszelle umfasst, wenn bestimmt wurde, dass die Methylierung der CpG-Stelle in der Promotorregion von mindestens einem der Gene AVPR1B und LRRC41 in dem Bestimmungsschritt vorhanden ist.

2. Verfahren nach Anspruch 1, wobei die Promotorregion eine Basensequenz von SEQ ID NO: 3 oder 4 umfasst.

3. Verfahren nach Anspruch 1 oder 2, wobei der Analyseschritt durch mindestens ein Verfahren durchgeführt wird, das aus Massenspektrometrie und methylierungsspezifischer PCR ausgewählt ist.

4. Verfahren nach einem der Ansprüche 1 bis 3, wobei die biologische Probe ein Gewebe, Blut oder Serum ist.

5. Verfahren nach einem der Ansprüche 1 bis 4, wobei
der Analyseschritt umfasst: Berechnen der Methylierungshäufigkeit der Promotorregion, und
der Schritt des Erhaltens umfasst: Vergleichen der Häufigkeit mit einer vorbestimmten Schwelle; und Erhalten von Informationen, dass die biologische Probe die Eierstockkrebszelle umfasst, wenn die Häufigkeit gleich oder höher als der Schwellenwert ist.

6. Verfahren nach einem der Ansprüche 1 bis 5, das zudem den Schritt Präparieren der DNA-Probe aus der biologischen Probe umfasst.

7. Verfahren nach Anspruch 6, wobei der Präparierschritt umfasst: Behandeln von DNA in der biologischen Probe mit Bisulfit.

8. Verwendung eines Reagenzkits in einem Verfahren zum Erhalten von Informationen, ob eine aus einem Individuum entnommene biologische Probe Eierstockkrebszellen enthält oder nicht, nach Anspruch 1, wobei der Kit einen Primer umfasst, wobei der Primer an eine Promotorregion von mindestens einem Gen, ausgewählt aus der Gruppe bestehend aus dem AVPR1B-Gen und dem LRRC41-Gen, hybridisieren kann, wobei die Promotorregion umfasst: eine Uracilbase und eine methylierte Cytosinbase, wobei die Uracilbase durch eine Bisulfit-Behandlung aus einer unmethylierten Cytosinbase umgewandelt wird.

9. Verwendung nach Anspruch 8, wobei der Primer irgendeine der Basensequenzen der SEQ ID NO: 7 bis 10 umfasst.

10. Verwendung nach Anspruch 8 oder 9, wobei der Primer zur Analyse des Methylierungszustands einer CpG-Stelle in einer Promotorregion von mindestens einem in der DNA-Probe enthaltenen Gen, ausgewählt aus der Gruppe bestehend aus dem AVPR1B-Gen und dem LRRC41-Gen, verwendet wird.

## Revendications

1. Procédé d'obtention d'une information selon laquelle un échantillon biologique collecté à partir d'un sujet contient ou non des cellules de cancer de l'ovaire, le procédé comprenant les étapes de :

   analyse de l'état de méthylation d'un site CpG dans une région de promoteur d'au moins un gène choisi dans le groupe constitué du gène AVPR1B et du gène LRRC41 contenus dans un échantillon d'ADN préparé à partir de l'échantillon biologique du sujet ; et
   obtention de l'information selon laquelle l'échantillon biologique comprend une cellule de cancer de l'ovaire lorsqu'il a été déterminé que la méthylation du site CpG dans la région de promoteur d'au moins un des gènes AVPR1B et LRRC41 est présent dans l'étape de détermination.

2. Procédé selon la revendication 1, dans lequel la région de promoteur comprend une séquence de bases de SEQ ID NO: 3 ou 4.

3. Procédé selon la revendication 1 ou 2, dans lequel l'étape d'analyse est conduite par au moins un procédé choisi parmi la spectrométrie de masse et la PCR spécifique à la méthylation.

4. Procédé selon l'une quelconque des revendications 1 à 3, dans lequel l'échantillon biologique est un tissu, du sang ou du sérum.

5. Procédé selon l'une quelconque des revendications 1 à 4, dans lequel
l'étape d'analyse comprend: le calcul de la fréquence de méthylation de la région de promoteur, et
l'étape d'obtention comprend : la comparaison de la fréquence à un seuil prédéterminé ; et l'obtention de l'information selon laquelle l'échantillon biologique comprend la cellule de cancer gynécologique lorsque la fréquence est égale ou supérieure au seuil.

6. Procédé selon l'une quelconque des revendications 1 à 5, qui comprend en outre l'étape de préparation de l'échantillon d'ADN à partir de l'échantillon biologique.

**7.** Procédé selon la revendication 6, dans lequel l'étape de préparation comprend : le traitement de l'ADN dans l'échantillon biologique avec du bisulfite.

**8.** Utilisation d'une trousse de réactif dans un procédé d'obtention de l'information selon laquelle un échantillon biologique collecté à partir d'un sujet contient ou non des cellules de cancer de l'ovaire selon la revendication 1, ladite trousse comprenant une amorce, dans laquelle l'amorce peut s'hybrider à une région de promoteur d'au moins un gène choisi dans le groupe constitué du gène AVPR1B et du gène LRRC41, dans laquelle la région de promoteur comprend : une base uracile et une base cytosine méthylée, la base uracile étant convertie à partir d'une base cytosine non méthylée par un traitement par bisulfite.

**9.** Utilisation selon la revendication 8, dans laquelle l'amorce comprend l'une quelconque des séquences de bases de SEQ ID NO: 7 à 10.

**10.** Utilisation selon la revendication 8 ou 9,
dans laquelle l'amorce est utilisée pour analyser l'état de méthylation d'un site CpG dans une région de promoteur d'au moins un gène choisi dans le groupe constitué du gène AVPR1B et du gène LRRC41 contenus dans l'échantillon d'ADN.

FIG. 1A

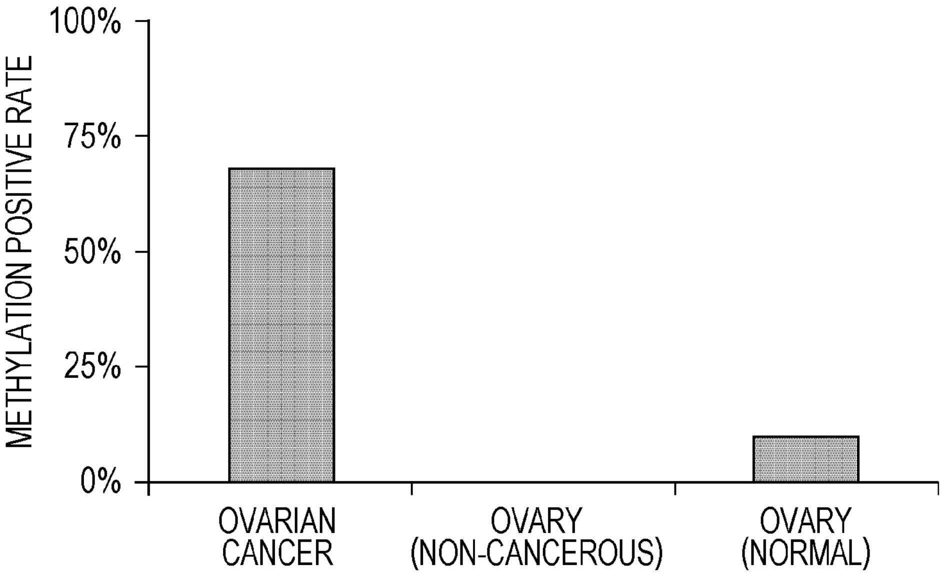
METHYLATION POSITIVE RATE
100%
75%
50%
25%
0%
OVARIAN CANCER
OVARY (NON-CANCEROUS)
OVARY (NORMAL)

FIG. 1B

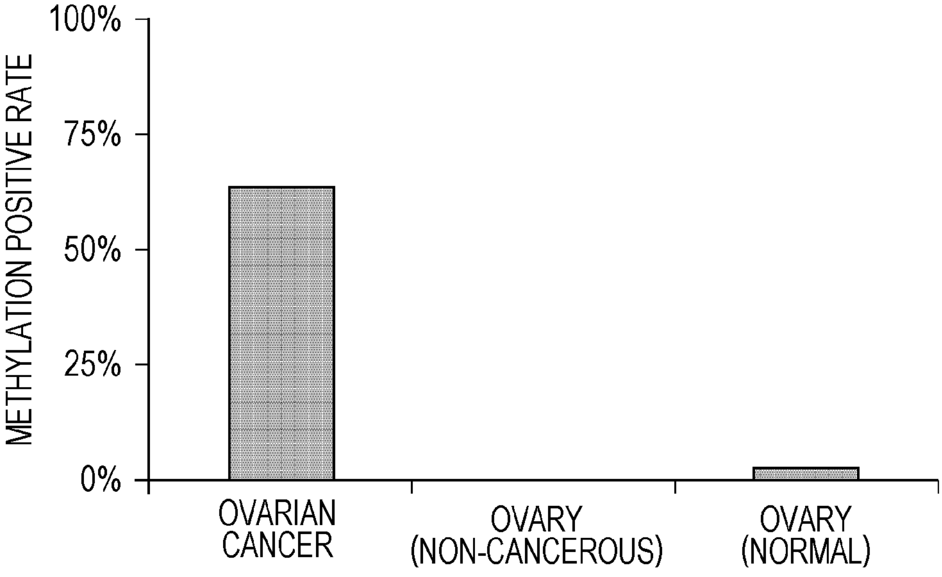
METHYLATION POSITIVE RATE
100%
75%
50%
25%
0%
OVARIAN CANCER
OVARY (NON-CANCEROUS)
OVARY (NORMAL)

FIG. 2A

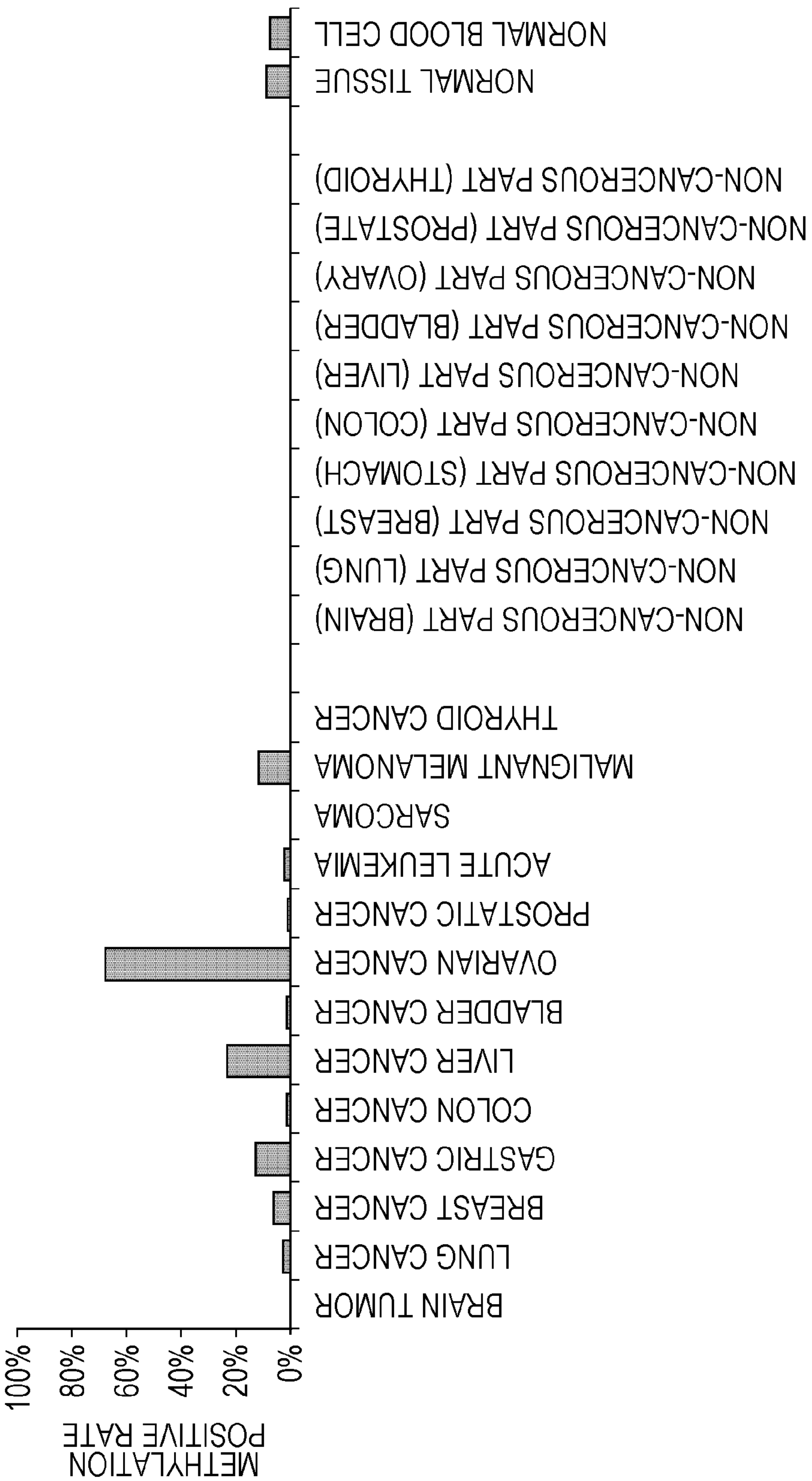
METHYLATION POSITIVE RATE
100%
80%
60%
40%
20%
0%
BRAIN TUMOR
LUNG CANCER
BREAST CANCER
GASTRIC CANCER
COLON CANCER
LIVER CANCER
BLADDER CANCER
OVARIAN CANCER
PROSTATIC CANCER
ACUTE LEUKEMIA
SARCOMA
MALIGNANT MELANOMA
THYROID CANCER
NON-CANCEROUS PART (BRAIN)
NON-CANCEROUS PART (LUNG)
NON-CANCEROUS PART (BREAST)
NON-CANCEROUS PART (STOMACH)
NON-CANCEROUS PART (COLON)
NON-CANCEROUS PART (LIVER)
NON-CANCEROUS PART (BLADDER)
NON-CANCEROUS PART (OVARY)
NON-CANCEROUS PART (PROSTATE)
NON-CANCEROUS PART (THYROID)
NORMAL TISSUE
NORMAL BLOOD CELL

FIG. 2B

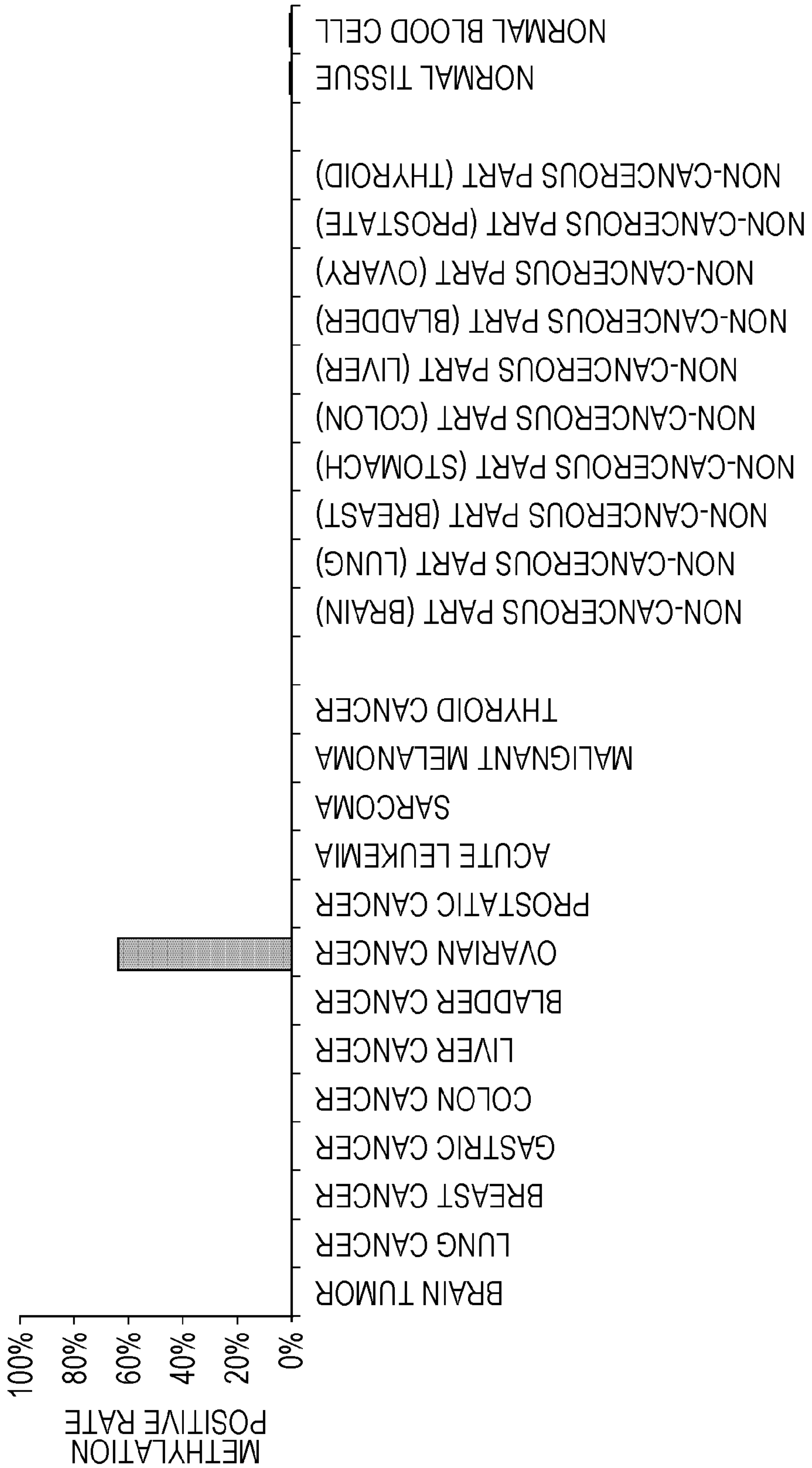
METHYLATION POSITIVE RATE
100%
80%
60%
40%
20%
0%
BRAIN TUMOR
LUNG CANCER
BREAST CANCER
GASTRIC CANCER
COLON CANCER
LIVER CANCER
BLADDER CANCER
OVARIAN CANCER
PROSTATIC CANCER
ACUTE LEUKEMIA
SARCOMA
MALIGNANT MELANOMA
THYROID CANCER
NON-CANCEROUS PART (BRAIN)
NON-CANCEROUS PART (LUNG)
NON-CANCEROUS PART (BREAST)
NON-CANCEROUS PART (STOMACH)
NON-CANCEROUS PART (COLON)
NON-CANCEROUS PART (LIVER)
NON-CANCEROUS PART (BLADDER)
NON-CANCEROUS PART (OVARY)
NON-CANCEROUS PART (PROSTATE)
NON-CANCEROUS PART (THYROID)
NORMAL TISSUE
NORMAL BLOOD CELL

FIG. 3

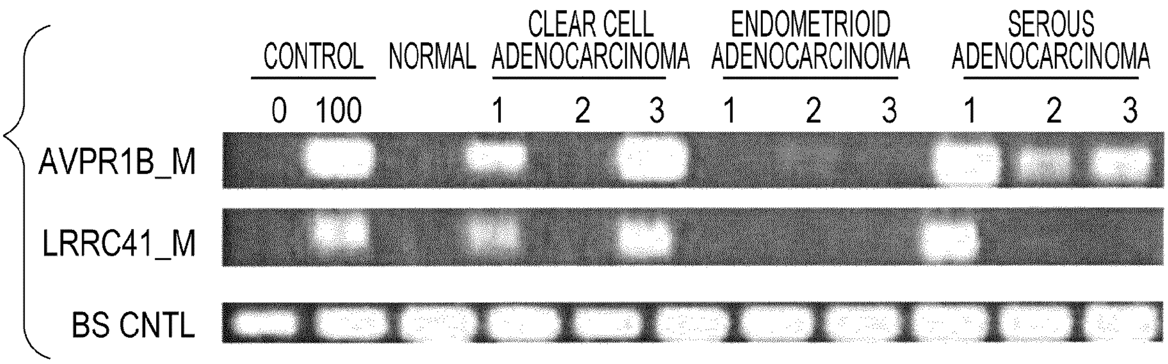
CONTROL
NORMAL
CLEAR CELL ADENOCARCINOMA
ENDOMETRIOID ADENOCARCINOMA
SEROUS ADENOCARCINOMA
0
100
1
2
3
1
2
3
1
2
3
AVPR1B_M
LRRC41_M
BS CNTL

FIG. 4

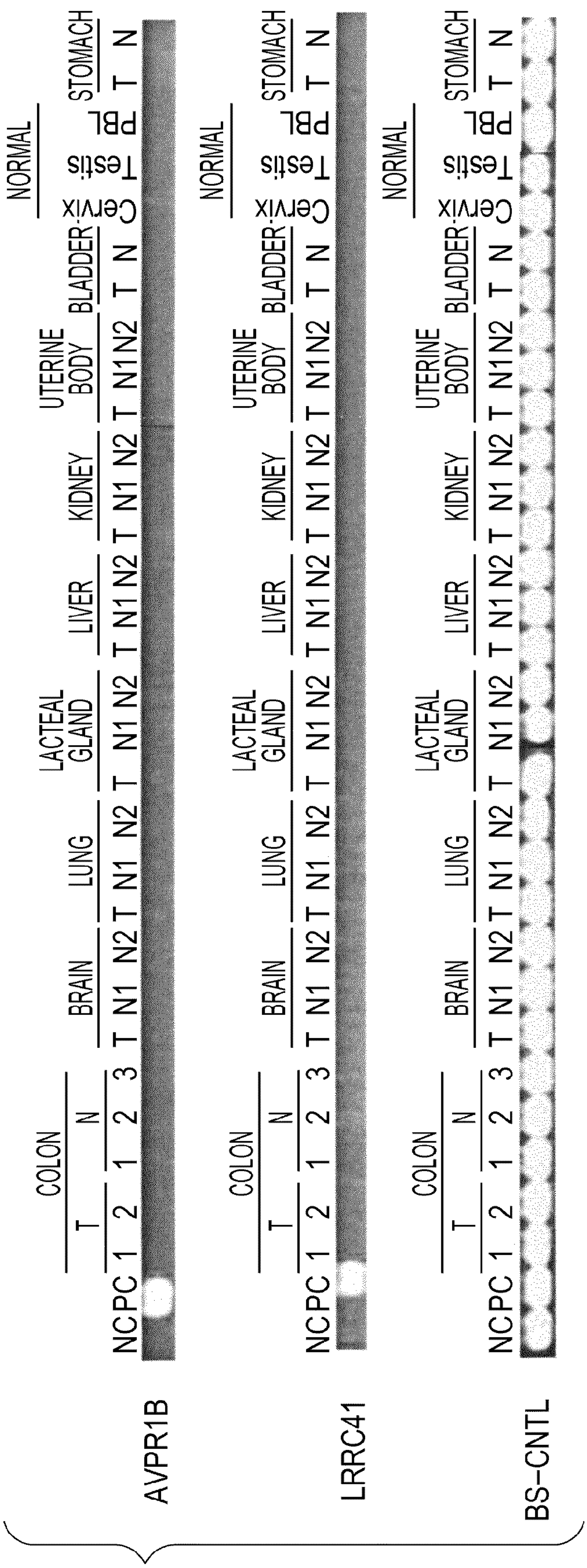
AVPR1B
LRRC41
BS-CNTL
NC PC
COLON
T
N
1 2 1 2 3
BRAIN
T N1 N2
LUNG
T N1 N2
LACTEAL GLAND
T N1 N2
LIVER
T N1 N2
KIDNEY
T N1 N2
UTERINE BODY
T N1 N2
BLADDER
T N
NORMAL
Cervix
Testis
PBL
STOMACH
T N

FIG. 5

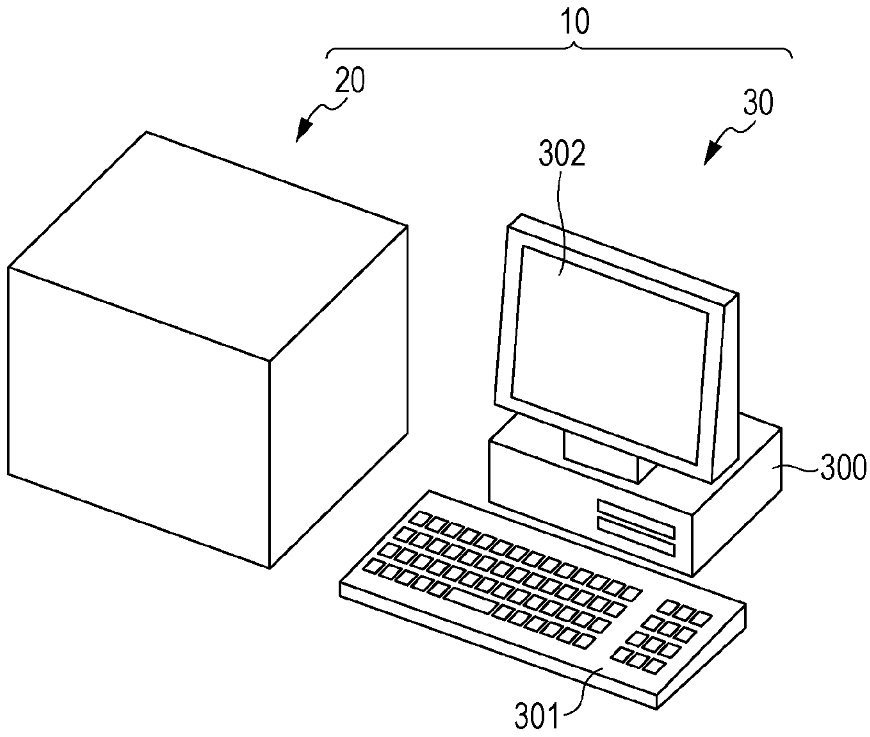
10
20
30
302
300
301

FIG. 6

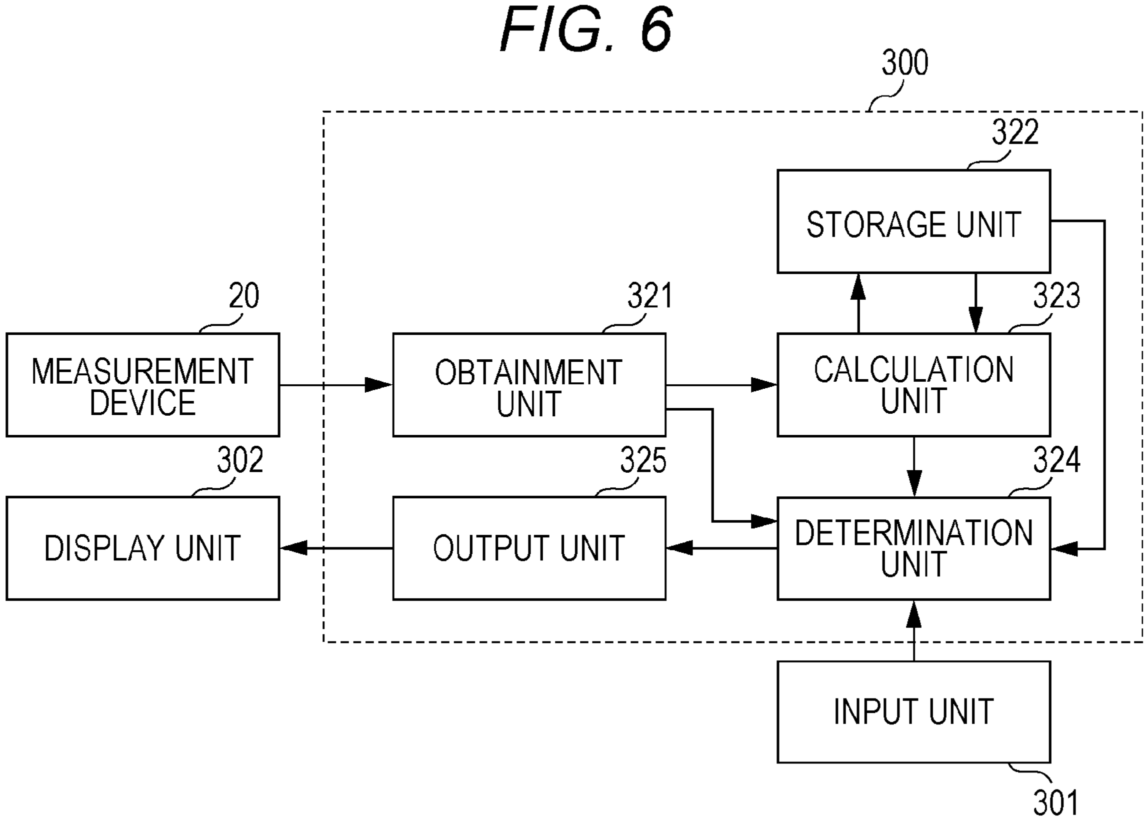
300
322
STORAGE UNIT
20
321
323
MEASUREMENT DEVICE
OBTAINMENT UNIT
CALCULATION UNIT
302
325
324
DISPLAY UNIT
OUTPUT UNIT
DETERMINATION UNIT
INPUT UNIT
301

FIG. 7

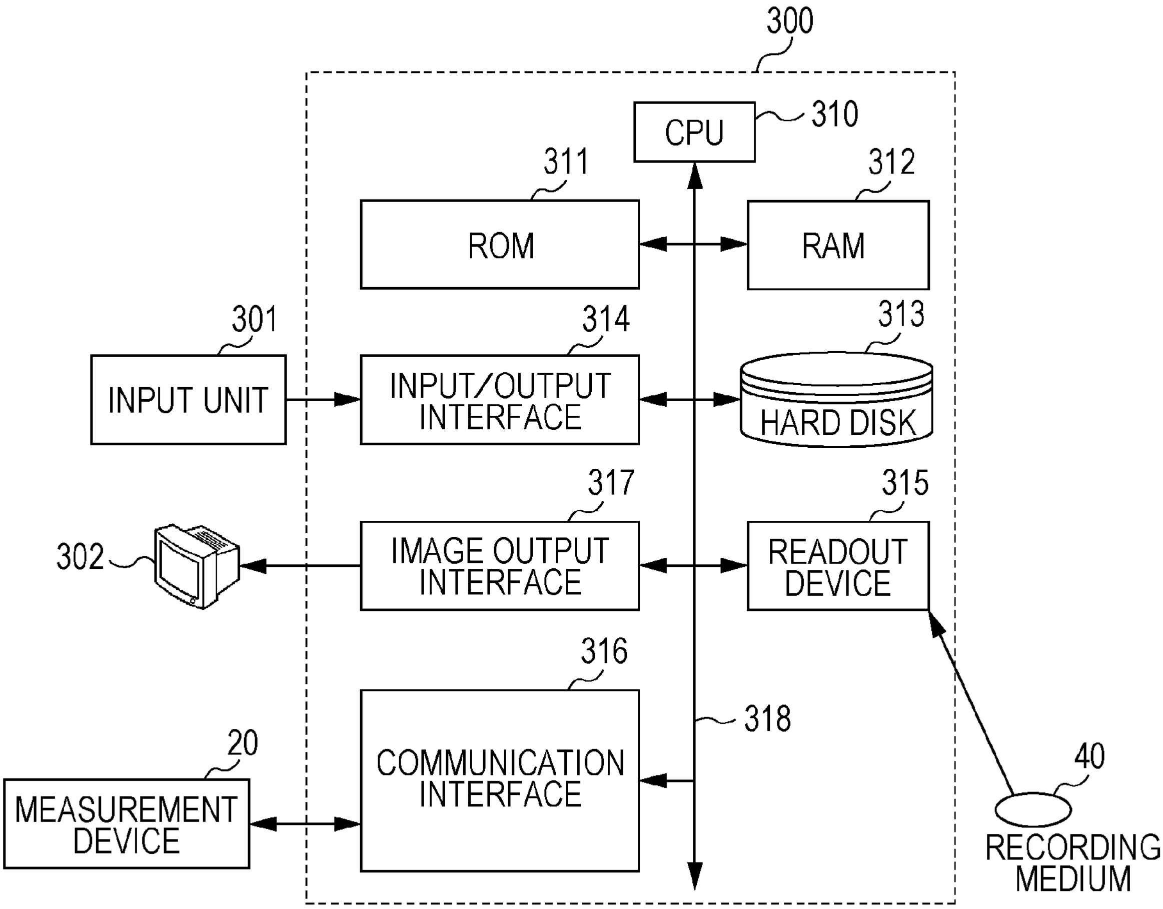
300
CPU
310
311
312
ROM
RAM
301
314
313
INPUT UNIT
INPUT/OUTPUT INTERFACE
HARD DISK
317
315
302
IMAGE OUTPUT INTERFACE
READOUT DEVICE
316
318
20
40
MEASUREMENT DEVICE
COMMUNICATION INTERFACE
RECORDING MEDIUM

FIG. 8

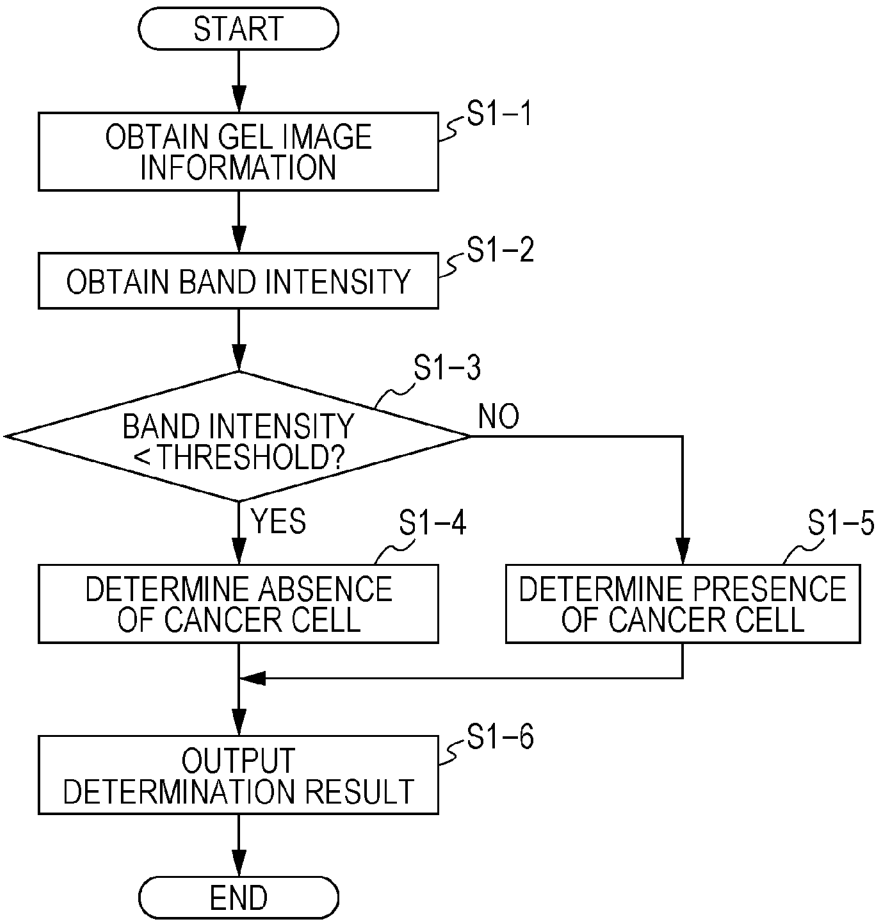
START
OBTAIN GEL IMAGE INFORMATION
S1-1
OBTAIN BAND INTENSITY
S1-2
BAND INTENSITY <THRESHOLD?
S1-3
NO
YES
DETERMINE ABSENCE OF CANCER CELL
S1-4
DETERMINE PRESENCE OF CANCER CELL
S1-5
OUTPUT DETERMINATION RESULT
S1-6
END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20110257034 A
- US 20130316931 A
- US 20100305058 A
- US 20070172844 A
- JP 2015079254 A


### Non-patent literature cited in the description


- **STEINBACH.** *Genomweite methylierungsanalysen und identifiziering prognostischer und prädiktiver marker fûr das epitheliale ovarialkarzinom,* 2014
- **KOLBE et al.** *PLOS One,* 2012, vol. 7 (3), e32941
- **NAZOR KL. et al.** Recurrent variations in DNA methylation in human pluripotent stem cells and their differentiated derivatives. *Cell Stem Cell,* 2012, vol. 10 (5), 620-634
- **NAZOR KL et al.** Recurrent variations in DNA methylation in human pluripotent stem cells and their differentiated derivatives. *Cell Stem Cell,* 2012, vol. 10 (5), 620-634
- **REINIUS LE et al.** Differential DNA Methylation in Purified Human Blood Cells: Implications for Cell Lineage and Studies on Disease Susceptibility. *PLoS One,* vol. 7 (7), e41361